# FASCICULE DE BREVET EUROPEEN

(11) **EP 3 857 221 B1**
(45) Date de publication et mention de la délivrance du brevet: **08.04.2026**
(21) Numéro de dépôt: 19798118.6
(22) Date de dépôt: 30.09.2019
(51) Int. Cl.: G01N 33/497, G01N 33/00

(54) **APPAREIL PORTABLE DE MESURE DE LA CONCENTRATION D'AU MOINS UN COMPOSANT DANS UN GAZ EXHALE PAR UN FLUIDE D'HALEINE**
TRAGBARE VORRICHTUNG ZUR MESSUNG DER KONZENTRATION MINDESTENS EINER KOMPONENTE IN EINEM DURCH EIN ATEMFLUID AUSGEATMETEN GAS
PORTABLE DEVICE FOR MEASURING THE CONCENTRATION OF AT LEAST ONE COMPONENT IN A GAS EXHALED BY A BREATH FLUID

(30) Priorité: 28.09.2018 FR 1859043
(43) Date de publication de la demande: 04.08.2021
(73) Titulaire: Dräger Safety AG & Co. KGaA, 23560 Lübeck (DE)
(72) Inventeur: NESA, Guillaume, 13510 EGUILLES (FR); FLESCH, Etienne, 78570 ANDRESY (FR)
(74) Mandataire: Guthöhrlein, Gerhard
(86) Numéro de dépôt international: PCT/FR2019/052304
(87) Numéro de publication internationale: WO 2020/065241

(56) Documents cités:
- WO-A1-2011/143693
- FR-B1- 2 730 314
- US-A- 4 649 027
- US-A1- 2003 052 692
- US-A1- 2016 338 620

## Description

### Domaine technique de l'invention.

L'invention a pour objet un appareil portable de mesure de la concentration d'au moins un composant dans un gaz exhalé par un fluide d'haleine. Elle concerne également un procédé de fonctionnement et un procédé d'utilisation d'un tel appareil.

L'invention concerne le domaine technique des dispositifs électroniques portables, tels que les éthylomètres ou les éthylotests par exemple, pour mesurer ou détecter la concentration d'un composant d'un gaz exhalé par un fluide d'haleine.

### État de la technique.

On connait par le document brevet FR2730314B1 (SERES), un appareil portable de mesure de la concentration d'au moins un composant dans un gaz exhalé par un fluide d'haleine comportant :
- un embout au travers duquel le fluide d'haleine est exhalé,
- un boîtier intégrant :
   ∘ une cuve de mesure,
   ∘ un moyen de mesure adapté à la mesure de la concentration d'au moins un composant dans un gaz du fluide d'haleine circulant dans la cuve de mesure,
   ∘ une ouverture dans laquelle est installé l'embout.

L'embout comporte :
- une première chambre comprenant un orifice d'entrée au travers duquel le fluide d'haleine exhalé pénètre dans ladite première chambre,
- une seconde chambre adjacente à la première chambre, laquelle seconde chambre comporte :
   ∘ un orifice d'entrée débouchant dans la première chambre,
   ∘ un premier orifice de sortie en communication fluidique avec la cuve de mesure et au travers duquel passe une partie du fluide d'haleine exhalé,
   ∘ un second orifice de sortie à l'air ambiant, et au travers duquel la partie du fluide d'haleine qui ne circule pas dans la cuve de mesure, est expulsé à l'air ambiant.

L'orifice d'entrée de la première chambre et le second orifice de sortie à l'air ambiant de la seconde chambre sont alignés sur un même axe et ont sensiblement la même section. Lorsque les pressions de souffle sont importantes, l'expulsion de l'air par le second orifice de sortie à l'air ambiant, crée des dépressions par effet Venturi au niveau du premier orifice de sortie de la seconde chambre. Cela se traduit par une aspiration locale de l'air présent dans la cuve de mesure. La mesure de concentration d'un composant d'intérêt dans le fluide d'haleine exhalé s'en trouve alors fortement affectée et peut être non représentative de la quantité réelle du composant d'intérêt présent dans ledit fluide.

Pour pallier ce problème, la solution mise en œuvre sur la plupart des appareils portables de mesure connus de l'état de l'art consiste à utiliser un moyen de pompage capable de créer une dépression importante en sortie de cuve de mesure, et ce afin de compenser cet effet Venturi. Outre le coût d'un tel composant, cette solution est contraignante lorsque l'on souhaite miniaturiser davantage lesdits appareils et réduire la taille des batteries utilisées. En effet, ce moyen de pompage est une source non négligeable de consommation d'énergie et demeure relativement encombrant.

L'invention vise à remédier à cet état des choses. En particulier, un objectif de l'invention est de garantir la fiabilité et la précision de mesure de l'appareil de mesure, tout en simplifiant sa conception et en réduisant son coût de fabrication.

Un autre objectif de l'invention est de proposer un appareil de mesure dont la conception le rend particulièrement compact par rapport aux appareils connus de l'art antérieur.

On connait également par le document brevet US2017/0100057 (WANG), un appareil où le boîtier présente un logement débouchant au niveau d'une ouverture dans laquelle est engagé l'embout.

Dans ce type d'appareil, l'installation des composants électroniques (ex : moyen de mesure, moyen de pompage, unité de commande) dans le boîtier est généralement coûteuse en temps et en main d'œuvre. En effet, le boîtier peut être de taille réduite de sorte que la mise en place des composants, leur fixation et leur connexion est mal aisée.

En outre, lorsqu'un des composants électroniques tombe en panne, son remplacement et/ou sa réparation sont problématiques. En effet, il convient de démonter le boîtier, de tester les composants pour diagnostiquer la panne, d'effectuer la réparation, de refermer le boîtier, et éventuellement d'étalonner de nouveau l'appareil. La mise en œuvre de toutes ces étapes peut être fastidieuse et coûteuse en temps. En outre, elle nécessite la neutralisation complète de l'appareil, c'est-à-dire qu'il n'est pas utilisable.

Face à cet état des choses, un objectif subsidiaire de l'invention est de réduire le temps de montage de différents composants électroniques de l'appareil et de faciliter l'installation de ces composants électroniques dans le boîtier.

Un autre objectif subsidiaire de l'invention est de proposer un appareil dont la conception permet d'intervenir sur un composant électronique défectueux, tout en réduisant la durée de neutralisation de l'appareil.

### Divulgation de l'invention.

La solution proposée par l'invention est un appareil portable de mesure de la concentration d'au moins un composant dans un gaz exhalé par un fluide d'haleine selon le préambule de le revendication 1 et présentant également les caractéristiques de la partie caractérisante de la revendication 1.

De par la conception de l'appareil selon l'invention,

l'expulsion à l'air ambiant de la partie du fluide d'haleine est maintenant réalisée au niveau de la première chambre et non pas au niveau de la seconde chambre. On s'assure ainsi qu'il n'y ait pas de dépression par effet Venturi au niveau de l'orifice de sortie de la seconde chambre qui communique avec la cuve de mesure. Il n'y a aucune aspiration locale de l'air présent dans cette cuve de sorte que les mesures sont fiables et précises. En outre, il n'est plus nécessaire de prévoir un moyen de pompage pour créer une dépression en sortie de cuve de mesure. Sans ce composant, l'encombrement de l'appareil peut être réduit et la consommation d'énergie électrique diminuée.

D'autres caractéristiques avantageuses de l'invention sont listées ci-dessous. Chacune de ces caractéristiques peut être considérée seule ou en combinaison avec les caractéristiques remarquables définies ci-dessus, et faire l'objet, le cas échéant, d'une ou plusieurs demandes de brevet divisionnaires :
- Selon un mode de réalisation, la première chambre et la seconde chambre sont réalisées dans l'embout.
- Selon une variante de réalisation, la première chambre et la seconde chambre sont réalisées dans le boîtier.
- Selon une autre variante de réalisation, la cuve de mesure, le moyen de mesure, un moyen de pompage adapté pour extraire le fluide d'haleine circulant dans la cuve de mesure et l'unité de commande sont réunis sur un support commun de manière à former un ensemble unitaire préhensible, lequel ensemble est installé de manière amovible dans le logement du boîtier ; la première chambre et la seconde chambre sont réalisées dans le support commun.
- Selon un mode de réalisation, la seconde chambre a des dimensions réduites par rapport à celles de la première chambre.
- Selon un mode de réalisation, la cuve de mesure, le moyen de mesure, un moyen de pompage et l'unité de commande sont réunis sur un support commun de manière à former un ensemble unitaire préhensible, lequel ensemble est installé de manière amovible dans le logement du boîtier.
- Selon un mode de réalisation, le boîtier est formé d'au moins deux tubes allongés présentant un axe longitudinal commun, lesquels tubes s'emboîtent selon ledit axe longitudinal pour définir le logement ; l'ensemble unitaire est installé dans un des tubes, ce tube formant un porte-embout dans lequel s'emboîte l'embout.
- Selon un mode de réalisation, l'autre tube formant le boîtier est adapté pour recevoir une batterie électrique adaptée pour alimenter l'ensemble unitaire préhensible.
- Selon un mode de réalisation, le support commun est adapté pour assurer une communication fluidique entre la cuve de mesure et l'orifice de sortie de la seconde chambre.
- Selon un mode de réalisation, le support commun comporte : - un logement dans lequel est installée la cuve de mesure ; - au moins un logement dans lequel est installé le moyen de mesure ; - un logement dans lequel est installé le moyen de pompage ; - un ou plusieurs aménagements adaptés pour recevoir l'unité de commande.
- Selon un mode de réalisation, le support commun comporte : - un premier perçage qui débouche dans un logement dans lequel est installée la cuve de mesure de sorte que ledit perçage soit en communication fluidique avec ladite cuve ; - un second perçage qui débouche dans une chambre aménagée dans ledit support et dans laquelle est installé un capteur de pression ; - l'orifice de sortie de la seconde chambre est en communication fluidique avec le premier perçage et avec le second perçage.
- Selon un mode de réalisation, le premier perçage est conique, lequel premier perçage comporte un premier orifice et un second orifice qui débouche dans le logement dans lequel est installée la cuve de mesure, le diamètre dudit premier orifice étant inférieur au diamètre dudit second orifice.
- Selon un mode de réalisation, l'orifice de sortie à l'air ambiant est dimensionné de sorte que 80% à 98% du fluide d'haleine exhalé dans la première chambre soit expulsé à l'air ambiant.
- Selon un mode de réalisation : - l'orifice d'entrée de la première chambre, l'orifice d'entrée de la seconde chambre, et l'orifice de sortie de ladite seconde chambre, sont disposés dans le même alignement ; - l'orifice de sortie à l'air ambiant est orienté selon une direction qui est perpendiculaire à cet alignement.
- Selon un mode de réalisation, le logement dans lequel est installé le moyen de pompage comporte un perçage débouchant dans le logement dans lequel est installée la cuve de mesure de sorte que lesdits logements soient en communication fluidique.
- Selon un mode de réalisation : - la cuve de mesure et le boîtier présentent chacun un axe longitudinal, lesquels axes sont parallèles ; - un moyen de pompage est configuré pour expulser le fluide d'haleine circulant dans la cuve de mesure selon une direction parallèle auxdits axes longitudinaux.
- Selon un mode de réalisation, comporte deux bordures longitudinales opposées qui sont solidarisées l'une à l'autre par collage pour maintenir la conformation dudit support sous forme de tube ; - une desdites bordures présente une bande dépourvue d'élément résistif chauffant.
- Selon un mode de réalisation, le ou les filaments chauffants recouvrent le support flexible souple de manière homogène de sorte que la densité de puissance électrique développée par l'élément résistif chauffant est identique sur toute la seconde face dudit support.
- Selon une variante de réalisation, le ou les filaments chauffants recouvrent le support flexible souple de manière non homogène de sorte que la densité de puissance électrique développée par l'élément résistif chauffant varie selon un axe longitudinal et/ou selon un axe transversal du support flexible souple.
- Selon un mode de réalisation, les filaments chauffants forment des sous-ensembles résistifs chauffants connectés électriquement en parallèle.
- Selon un mode de réalisation : - la cuve de mesure se présente sous la forme d'un tube ouvert à ses deux extrémités ; - le moyen de mesure comporte un émetteur de rayonnement infrarouge monté au niveau d'une extrémité de la cuve de mesure de manière à ce que ladite cuve soit traversée par un rayonnement infrarouge, et un détecteur de rayonnement infrarouge monté à l'autre extrémité de la cuve de mesure ; - une cavité étanche au fluide d'haleine est interposée entre l'émetteur de rayonnement infrarouge et l'extrémité correspondante de la cuve de mesure ; - une cavité étanche au fluide d'haleine est interposée entre le détecteur de rayonnement infrarouge et l'extrémité correspondante de la cuve de mesure.
- Selon un mode de réalisation, ledit appareil est un éthylomètre ou un éthylotest.

Un autre aspect de l'invention concerne un procédé de régulation de la température de la cuve de mesure de l'appareil, consistant à réguler l'énergie électrique injectée dans l'élément résistif chauffant grâce à une boucle de contre-réaction basée sur : la mesure en temps réel de la résistance dudit élément et l'objectif d'atteindre une résistance de consigne correspondant à une température de chauffage cible.

Un autre aspect de l'invention concerne un procédé d'utilisation de l'appareil comprenant les étapes consistant à :
- enregistrer et associer, dans une base de données, un moyen d'identification de l'appareil et un moyen d'identification d'un utilisateur,
- préalablement à la mesure, acquérir, depuis un terminal mobile de l'utilisateur, le moyen d'identification de l'appareil et le moyen d'identification de l'utilisateur,
- analyser le moyen d'identification de l'appareil acquis et le moyen d'identification de l'utilisateur acquis,
- seulement en cas de correspondance entre le moyen d'identification de l'appareil acquis et le moyen d'identification de l'utilisateur acquis, transmettre à l'unité de commande une instruction pour opérer la mesure, laquelle instruction est générée depuis le terminal mobile.

L'acquisition du moyen d'identification de l'utilisateur est avantageusement basée sur la mise en œuvre d'un algorithme de reconnaissance faciale dudit utilisateur.

L'acquisition du moyen d'identification de l'appareil peut être basée sur la mise en œuvre d'un algorithme de reconnaissance de la forme dudit appareil ou sur la mise en œuvre d'un algorithme de reconnaissance d'un marquage apposé sur ledit appareil.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1a est une vue en perspective avant d'un appareil conforme à l'invention,
- la figure 1b est une vue en perspective arrière de l'appareil de la figure 1a,
- la figure 2a est une vue éclatée en perspective avant de l'appareil des figures 1a et 1b,
- la figure 2b est une vue éclatée en perspective arrière de l'appareil des figures 1a et 1b,
- la figure 3a est une vue en perspective avant d'un ensemble unitaire selon un premier mode de réalisation,
- la figure 3b est une vue en perspective arrière de l'ensemble unitaire de la figure 3a,
- la figure 4 est une vue en perspective d'un support commun selon un premier mode de réalisation,
- la figure 5 est une vue éclatée en perspective du support commun de la figure 4 avec une cuve de mesure conformée sous forme de tube,
- la figure 6 est une vue en coupe longitudinale du support commun de la figure 4 et sur lequel sont montés le moyen de mesure et le moyen de pompage,
- la figure 7 est une vue en perspective d'un embout adapté pour coopérer avec le support commun des figures 4 à 6,
- la figure 8 est une vue en coupe longitudinale de l'embout de la figure 7,
- la figure 9 est une vue en coupe transversale de l'embout des figures 7 et 8 et du support commun des figures 4 à 6, montés dans le boîtier,
- la figure 10 est une vue en perspective d'un support commun selon un deuxième mode de réalisation,
- la figure 11 est une vue en coupe longitudinale du support commun de la figure 10 et sur lequel sont montés le moyen de mesure et le moyen de pompage,
- la figure 12 est une vue en perspective d'un embout adapté pour coopérer avec le support commun des figures 10 et 11,
- la figure 13 est une vue en coupe longitudinale de l'embout de la figure 12,
- la figure 14 est une vue en coupe transversale de l'embout des figures 12 et 13 et du support commun des figures 10 et 11, montés dans le boîtier,
- la figure 15 est une vue en coupe transversale d'un boîtier, selon un troisième mode de réalisation de l'invention,
- la figure 16 est une vue en coupe longitudinale d'un boîtier selon la figure 15,
- la figure 17 est une vue en coupe transversale du support commun des figures 4 à 6, monté dans le boîtier des figures 15 et 16,
- les figures 18a, 18c et 18d sont des vues de face de plusieurs variantes d'un support flexible souple formant la cuve de mesure, lequel support est à plat,
- la figure 18b est une vue de l'autre face du support des figures 18a, 18c et 18d,
- les figures 19a à 19i illustrent différentes interfaces graphiques visibles sur un terminal mobile appairé à un appareil conforme à l'invention,
- la figure 20 illustre de manière simplifiée la structure d'une unité de commande utilisée dans l'invention.

### Modes préférés de réalisation de l'invention.

L'appareil objet de l'invention est destiné à mesurer la concentration d'au moins un composant (éthanol et/ou acétone et/ou CO et/ou CO₂ et/ou H₂O et/ou ...) dans un gaz exhalé par un fluide d'haleine. Il est particulièrement, mais non exclusivement, adapté pour être utilisé comme éthylomètre ou comme éthylotest pour la détection et/ou le contrôle d'alcoolémie, par exemple avant de conduire un véhicule. Il peut également être utilisé par des personnes diabétiques pour évaluer de manière indirecte leur glycémie par une mesure de la concentration d'acétone dans leur fluide d'haleine. Dans la suite de la description, les expressions « *gaz exhalé par un fluide d'haleine* »*,* « *fluide d'haleine* » ou « *gaz du*/*de fluide d'haleine* » sont des synonymes.

L'appareil est portable en ce sens qu'il est autonome et de taille suffisamment réduite pour être mis dans une poche de vêtement par exemple.

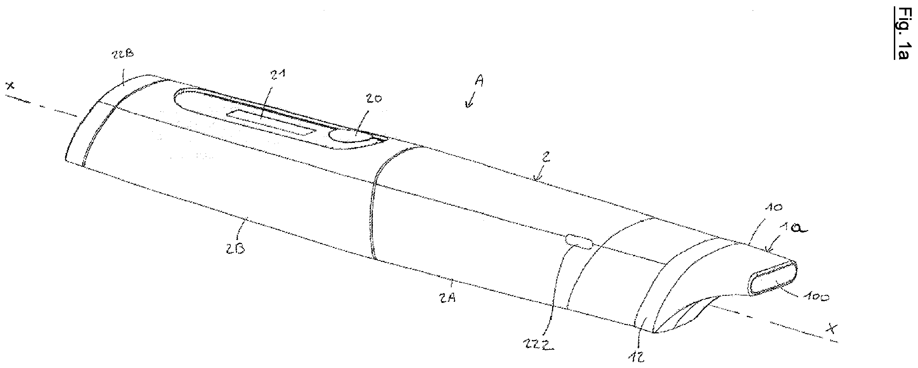

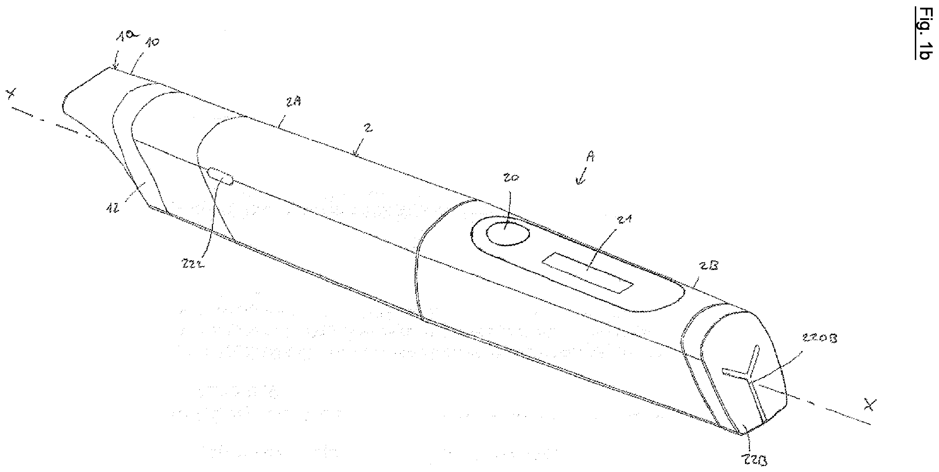

Sur les figures 1a et 1b, l'appareil A est de forme allongée et présente un axe longitudinal X-X. Il est avantageusement inscrit dans une enveloppe parallélépipédique dont la longueur est comprise entre 10 cm et 15 cm, une largeur comprise entre 1 cm et 3 cm et une hauteur comprise entre 1 cm et 3 cm. On peut donc considérer que l'appareil A est compact, avec un encombrement particulièrement réduit.

L'appareil A comporte un embout 1 au travers duquel le fluide d'haleine est exhalé par l'utilisateur. Sur les figures annexées, cet embout 1 a une forme en bec de flûte. Il est assemblé au niveau d'une extrémité d'un boîtier 2.

L'embout 1 et le boîtier 2 sont réalisés dans un matériau rigide, par exemple en plastique synthétique ou biosourcé (ex : PVC, ABS, PC, PA, PLA, PHA, PHB, PBS), en carbone, en matériau composite, en acier, etc. Ils peuvent être obtenus par moulage, par extrusion, par impression ou par tout autre procédé convenant à l'homme du métier. Ils ne nécessitent pas de traitement de surface particulier.

Sur les figures 1a et 1b, le boîtier 2 comporte, sur sa paroi, et de manière accessible, un bouton actionnable 20 pour la mise en marche/arrêt de l'appareil A et un moyen d'information 21 adapté pour afficher la valeur de la concentration du ou des composants du gaz mesurée. Ce moyen d'information 21 est préférentiellement un écran d'affichage du type écran OLED. Le moyen d'information 21 pourrait également consister en un haut-parleur indiquant de manière sonore la valeur mesurée et/ou un ou plusieurs voyants lumineux dont la couleur dépend de la valeur mesurée.

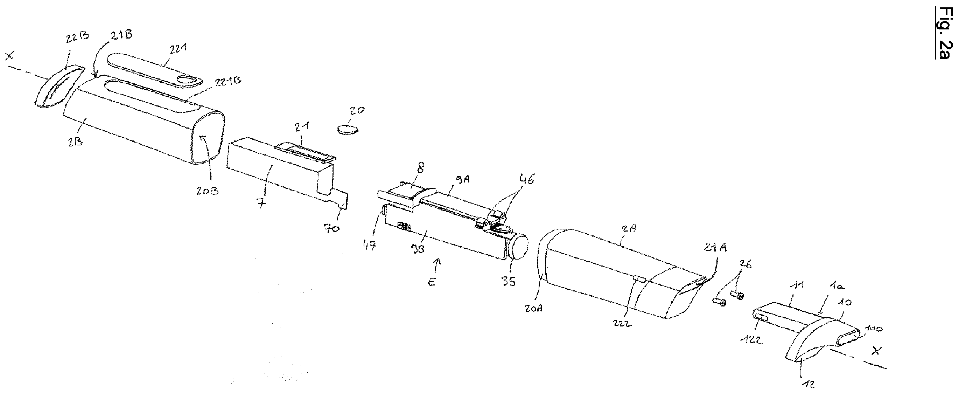

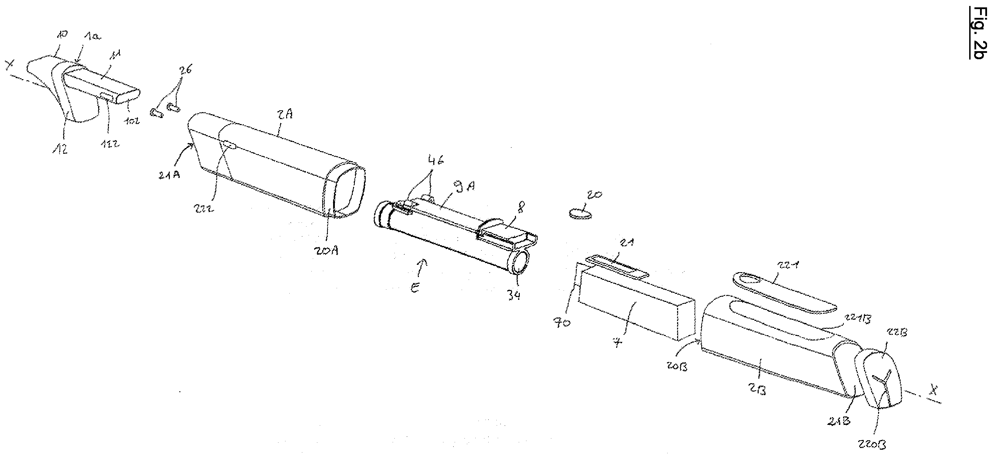

Le boîtier 2 présente un logement dans lequel sont installés les différents composants de l'appareil A. Sur les figures 2a et 2b, le boîtier 2 est formé de deux tubes allongés 2A, 2B ayant comme axe longitudinal commun, l'axe X-X. Ces deux tubes 2A, 2B s'emboîtent selon l'axe longitudinal X-X. Cette conception particulièrement simple a plusieurs avantages : elle permet de réaliser le boîtier 2 avec des formes simples et donc de réduire ses coûts de fabrication. En outre, le montage du boîtier 2 se fait très rapidement. Et enfin, l'assemblage/désassemblage des différents composants à l'intérieur du boîtier 2 peut également être réalisé de manière très rapide.

Les tubes 2A, 2B sont creux de sorte que leur paroi interne délimite le logement du boîtier 2. Ils peuvent avoir une section circulaire, carrée, rectangulaire, ovale, etc. Le tube avant 2A présente une extrémité ouverte 20A dont la paroi interne est conformée pour former un organe de connexion mâle. Et le tube arrière 2B présente une extrémité ouverte 20B complémentaire dont la paroi interne est conformée pour former un organe de connexion femelle pour accueillir de manière démontable l'organe de connexion mâle du tube avant 2A. Un ou plusieurs éléments d'encliquetage peuvent être prévus pour assurer un maintien en position efficace des deux tubes 2A, 2B. Une solution par vissage ou collage (par exemple par adhésif) des tubes 2A, 2B peut également être envisagée. Comme expliqué plus avant dans la description, les deux tubes 2A, 2B délimitent un logement dans lequel sont installés les différents composants de l'appareil A. L'autre extrémité ouverte 21B du tube arrière 2B est obturée par un bouchon 22B, lequel bouchon comporte une ouverture 220B mettant en communication fluidique l'intérieur dudit tube avec l'air ambiant.

En se rapportant à la figure 2a, le tube avant 2A présente une ouverture 21A dans laquelle s'emboîte l'embout 1a. Le tube avant 2A fait ainsi office de porte-embout. Il est à noter que le logement du boîtier 2 débouche au niveau de cette ouverture 21A. Pour des raisons d'hygiène, l'embout 1a est avantageusement jetable de sorte qu'il est déconnectable du boîtier 2, et plus particulièrement de l'ouverture 21A. Le fait que l'embout 1a soit un consommable nécessite que sa forme soit la plus simple possible, et son poids minimal afin de réduire au maximum les coûts du procédé de fabrication et de matière de ces embouts. Certaines utilisations particulières du dispositif de mesure pour des applications médicales ou pour des usages intensifs peuvent nécessiter l'emploi de matériaux biocompatibles ou biodégradables pour des questions environnementales ou normatives. L'ensemble de ces contraintes peuvent donc être prises en compte dans la conception des tels embouts.

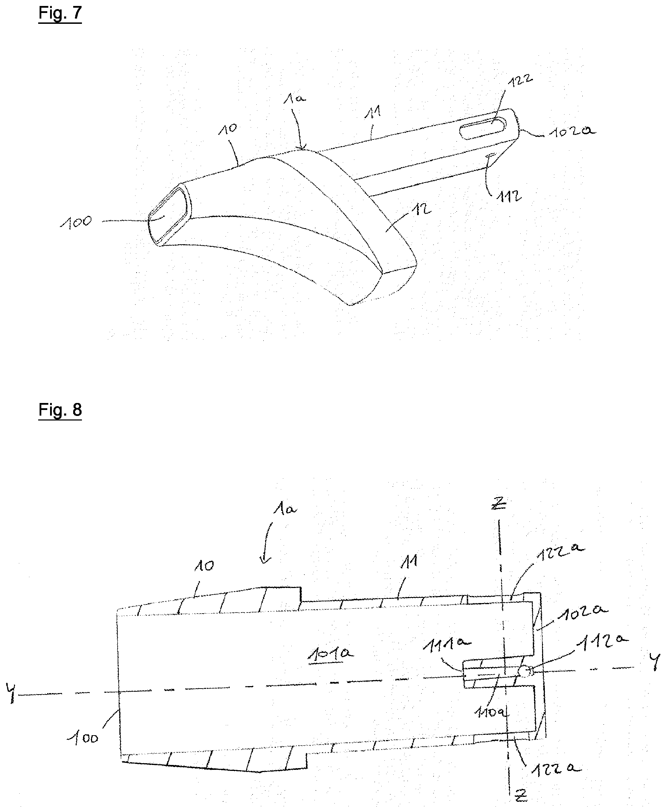

Sur les figures 2a, 2b et 7, l'embout 1a présente un bec 10 pourvu d'un orifice d'entrée 100 au travers duquel le fluide d'haleine est exhalé. En pratique, l'utilisateur pose ses lèvres sur le bec 10 et expire au travers de l'orifice d'entrée 100. Ce dernier à une forme oblongue dont la largeur correspond sensiblement à celle de l'appareil A. La hauteur de l'orifice d'entrée 100 est par exemple comprise entre 1 mm et 10 mm. Le bec 10 se prolonge, selon l'axe X-X, par une âme 11 dont la section correspond sensiblement à celle de l'orifice d'entrée 100. C'est cette âme 11 qui s'engage dans l'ouverture 21A du tube avant 2A. L'âme 11 a par exemple une longueur comprise entre 10 mm et 30 mm. L'embout 1a présente également une jupe 12 aménagée à l'interface entre le bec 10 et l'âme 11, laquelle jupe vient en recouvrement de l'extrémité du tube avant 2A portant l'ouverture 21A. Cette jupe 12 sert également de moyen de préhension pour retirer l'embout 1a sans toucher la zone du bec 10 qui a été en contact avec les lèvres de l'utilisateur.

Conformément à l'invention, deux chambres sont disposées en amont d'une cuve de mesure 3 installée dans le boîtier 2.

### Premier mode de réalisation.

Selon un premier mode de réalisation, les deux chambres sont réalisées dans l'embout 1a.

Sur la figure 8, le bec 10 et l'âme 11 délimitent une première chambre 101a dans laquelle débouche l'orifice d'entrée 100. Le fluide d'haleine exhalé circule donc dans la première chambre 101a. Une paroi de fond 102a, disposée en vis-à-vis de l'orifice d'entrée 100, vient fermer la première chambre 101a.

Une seconde chambre 110a est disposée à l'intérieur de la première chambre 101a. Cette seconde chambre 110a a préférentiellement des dimensions réduites par rapport à celles de la première chambre 101a. Alors que la longueur de la première chambre 101a correspond à la longueur combinée du bec 10 et de l'âme 11, celle de la seconde chambre 110a ne correspond qu'à une fraction (ex : 1/7) de cette longueur. Il en est de même pour la largeur de seconde chambre 110a qui ne correspond qu'à une fraction (ex : 1/3) de celle de la première chambre 101a.

La seconde chambre 110a comporte un orifice d'entrée 111a, qui débouche dans la première chambre 101a, et au travers duquel passe une partie du fluide d'haleine exhalé circulant dans ladite première chambre. L'orifice d'entrée 111a a la même section, ou sensiblement la même section, que celle de la seconde chambre 110a. La paroi de fond 102a, disposée en vis-à-vis de l'orifice d'entrée 111a vient également fermer la seconde chambre 110a. La partie du fluide d'haleine exhalé circulant dans la seconde chambre 110a est extraite de l'embout 1a par un orifice de sortie 112a. Ce dernier est en communication fluidique avec la cuve de mesure 3 comme expliqué plus en avant dans la description. L'orifice de sortie 112a peut être de section circulaire, et a un diamètre qui correspond sensiblement à la largeur de la seconde chambre 110a. La surface de l'orifice de sortie 112a est inférieure à la surface de l'orifice d'entrée 100.

Les orifices 100, 111a et 112a sont disposés dans le même alignement Y-Y. Cet alignement est parallèle à l'axe longitudinal X-X précité. Cette configuration en ligne permet au fluide d'haleine soufflé dans l'embout 1a d'avoir un trajet direct entre l'entrée 100 et la sortie 112a, limitant de fait les pertes de charge. Par ailleurs, la distance séparant l'orifice d'entrée 100 de l'orifice de sortie 112a peut être relativement courte, notamment inférieure à 50 mm, de sorte que l'embout 1a est particulièrement compact.

L'autre partie du fluide d'haleine exhalé qui ne circule pas dans la seconde chambre 110a, est expulsée à l'air ambiant par au moins un orifice de sortie 122a débouchant dans la première chambre 101a. On prévoit préférentiellement deux orifices de sortie 122a qui sont réalisés dans la paroi de l'âme 11, près de la paroi de fond 102a. Ces orifices de sortie 122a sont des orifices latéraux, c'est-à-dire orientés selon une direction Z-Z qui est perpendiculaire à l'alignement Y-Y des orifices 100, 111a et 112a. Cette configuration particulière de l'embout 1a apporte un plus grand confort à l'utilisateur par rapport à des embouts connus de l'état de l'art pour lesquels l'orifice d'entrée du fluide d'haleine expiré et l'orifice de sortie (permettant à la fraction du fluide non utile à la mesure de concentration d'être expulsée à l'air ambiant) sont alignés sur un même axe. En effet, lorsque qu'une personne se trouve face à l'utilisateur, la fraction du fluide d'haleine exhalé qui est expulsée à l'air ambiant ne lui parvient pas en direct mais s'échappe en latéral, ce qui lui évite de le respirer.

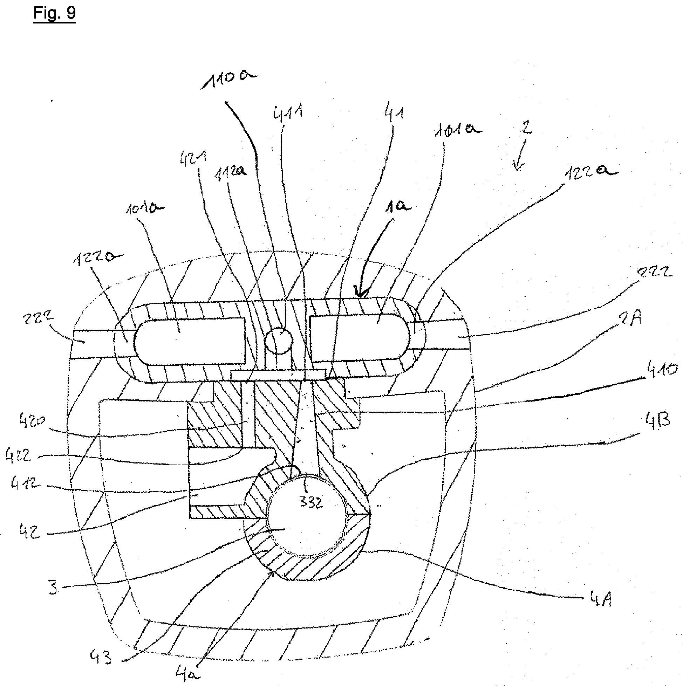

En se rapportant à la figure 9, lorsque l'embout 1a est installé dans l'ouverture 21A, les orifices de sortie 122a sont en vis-à-vis d'orifices 222 disposés latéralement sur le boîtier 2, et plus particulièrement sur le tube avant 2A. Ces orifices latéraux 222 sont également visibles sur les figures 1a, 1b, 2a et 2b.

Comme illustré sur la figure 8, l'entrée 111a de la seconde chambre 110a est avantageusement située en amont des orifices de sortie 122a. Et préférentiellement, les parois latérales de la seconde chambre 110a ont des dimensions selon l'axe Y-Y supérieures à celles des orifices de sortie 122a. Grâce à la position de l'entrée 111a et/ou de la longueur des parois latérales de la seconde chambre 110a, les turbulences du fluide exhalé créées dans la première chambre 101a, au niveau des orifices de sortie 122a, ne viennent pas perturber l'échantillonnage du gaz d'haleine dans ladite seconde chambre et, partant, dans la cuve 3. Le flux du fluide d'haleine échantillonné est globalement laminaire à partir de l'orifice d'entrée 111a de la seconde chambre 110a.

La surface combinée des orifices de sortie 122a est avantageusement inférieure à la surface de l'orifice d'entrée 100 de la première chambre 101a et supérieure à la surface de l'orifice de sortie 112a de la seconde chambre 110a, de sorte que seule une fraction minoritaire du fluide d'haleine passant au travers de l'orifice d'entrée 100 ressorte par l'orifice de sortie 112a, la fraction majoritaire dudit fluide étant expulsée à l'air ambiant via les orifices de sortie 122a.

De par la conception de l'embout 1a et de la configuration de ses différents orifices, lorsque l'utilisateur souffle au travers de l'orifice d'entrée 100 de la première chambre 101a, le fluide d'haleine exhalé est mis en pression dans ladite première chambre. Une partie du fluide d'haleine pénètre dans la seconde chambre 110a et en ressort par l'orifice de sortie 112a. Cet échantillon du fluide d'haleine qui sort par l'orifice de sortie 112a pénètre alors sous pression dans la cuve de mesure 3. Les orifices de sortie 122a, 222 créent un échappement qui réduit la pression de souffle nécessaire au bon fonctionnement de l'appareil A.

Pour la mesure de la concentration d'un composant dans le gaz de fluide d'haleine, le boîtier 2 intègre : une cuve de mesure 3 dans laquelle circule l'échantillon du fluide d'haleine sortant de l'embout 1a par l'orifice de sortie 112a ; un moyen de mesure 34, 35 ; éventuellement un moyen de pompage 8 adapté pour extraire le fluide d'haleine circulant dans la cuve de mesure ; une unité de commande 9 adaptée pour contrôler et piloter au moins le moyen de mesure 34,35, et, le cas échéant le moyen de pompage 8, et le moyen d'information 21. La mesure de la concentration est basée sur la loi de BEER LAMBERT bien connue de l'homme du métier.

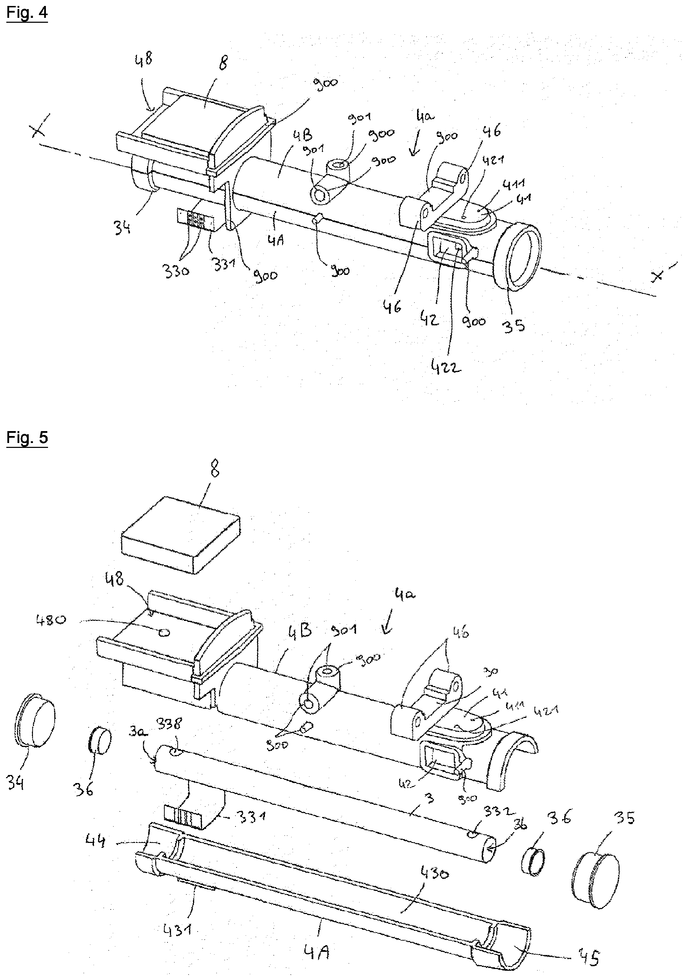

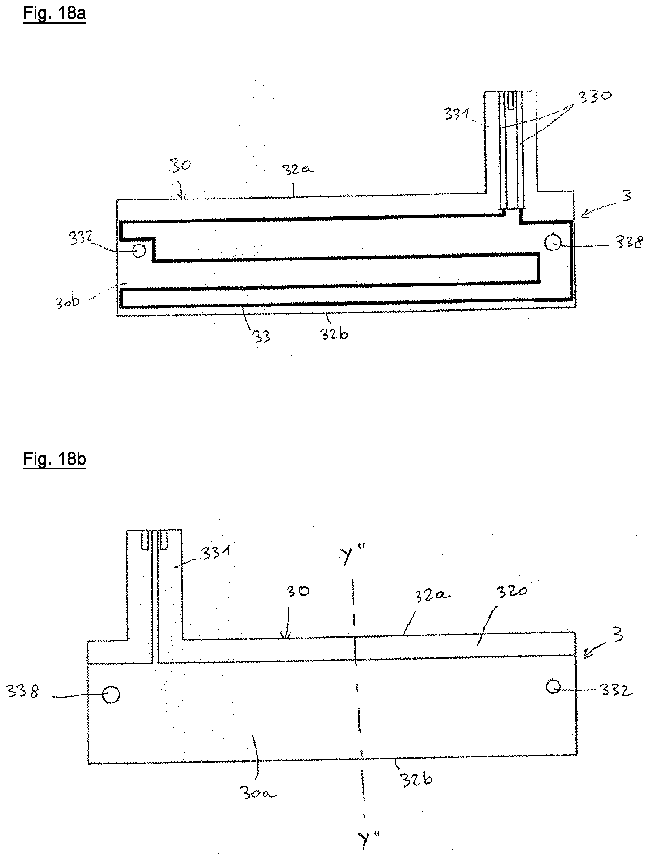

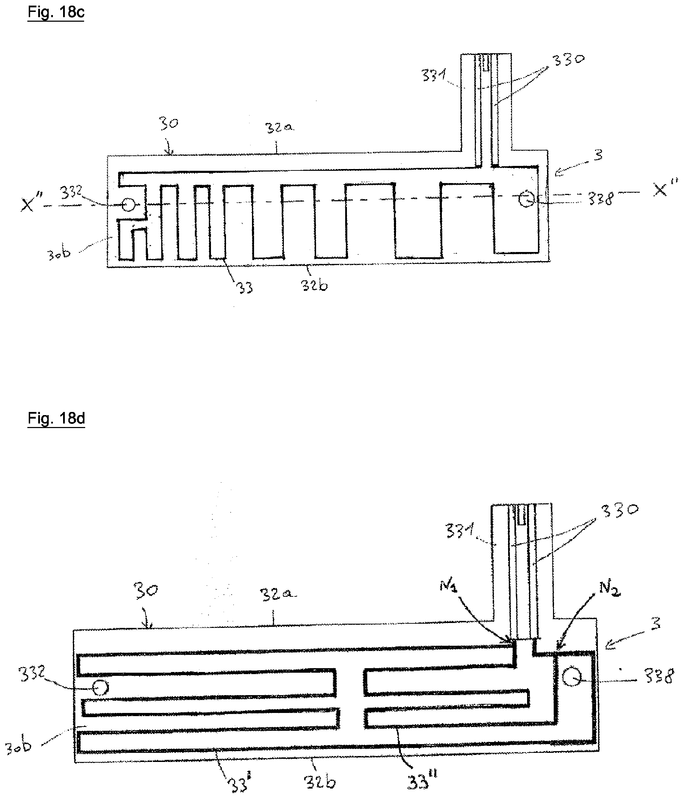

Sur les figures 5, 18a, 18b, 18c et 18d, la cuve de mesure 3 est réalisée à partir d'un support flexible souple 30 conformé sous la forme d'un tube, en accord avec l'invention.

Le support 30 consiste préférentiellement en un film mince ayant une épaisseur comprise entre 1 µm et 250 µm, préférentiellement environ 25 µm. Un bon rapport souplesse/résistance est obtenu avec ces valeurs d'épaisseur. Le support 30 est avantageusement réalisé dans un matériau choisi dans le groupe suivant : polyimide (par ex : Kapton^{®}), polyépoxyde, polyester, résine époxy renforcée de fibre de verre, substrat d'aluminium (par ex : support COOL-CLAD^{®} commercialisé par la société AI TECHNOLOGY). Tout autre matériau généralement utilisé pour la fabrication de circuit imprimé souple peut toutefois être envisagé. Le support 30 peut être obtenu par moulage, extrusion, laminage, etc.

Le support 30 comprend deux faces 30a, 30b qui sont opposées. Une des faces 30a du support 30 est recouverte d'un matériau métallique réfléchissant formant une couche de réflexion optique sur laquelle rebondira un rayonnement infrarouge émis. Cette couche de réflexion permet ainsi un guidage du rayonnement infrarouge émis. Pour que la couche de réflexion soit la plus réfléchissante possible et afin de limiter les pertes énergétiques du rayonnement émis, le matériau métallique réfléchissant est préférentiellement choisi dans le groupe suivant : or, cobalt, argent, nickel, cuivre, aluminium, chrome, zinc. Ce matériau métallique réfléchissant présente une épaisseur comprise entre 0,01 µm et 500 µm, préférentiellement entre 0,01 µm et 10 µm. Il peut être déposé par collage, par dépôt électrochimique, par dépôt électrolytique, par impression, par sérigraphie, chauffage, ou par tout autre procédé de dépôt de couche mince. Dans le but d'assurer un bon maintien en position du matériau métallique réfléchissant sur la face 30a du support 30, une ou plusieurs couches d'accroche peuvent être déposées sur cette face. Il s'agit par exemple de couches de matériaux tels que le cuivre, l'aluminium, l'argent, le nickel, le palladium, le polyéthylène ou une combinaison de ces matériaux, dont l'épaisseur totale est typiquement comprise entre 0,1 µm et 500 µm, préférentiellement entre 0,1 µm et 200 µm, déposée par un procédé de dépôt de couche mince. La couche d'accroche n'est pas indispensable et peut notamment être évitée dans le cas où le matériau métallique réfléchissant est déposé, par exemple, par dépôt électrolytique.

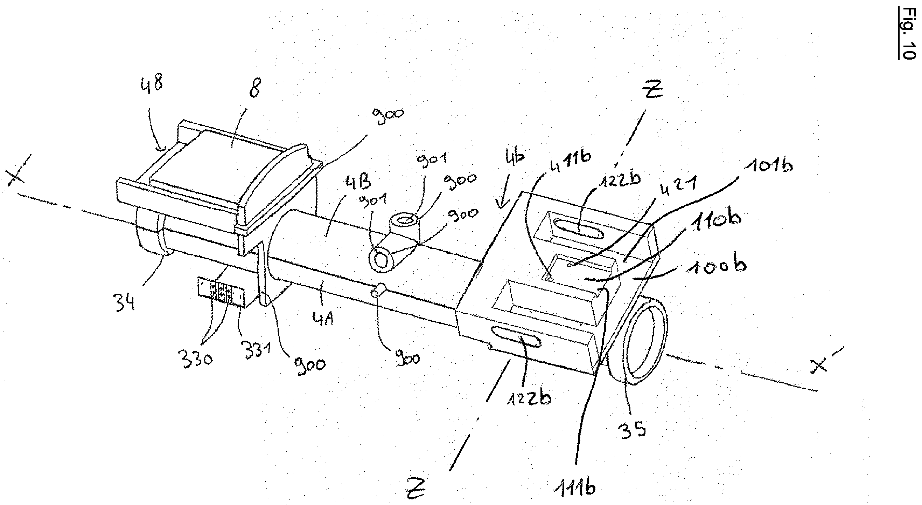

Un élément résistif chauffant 33 flexible est disposé sur au moins une des faces 30a et/ou 30b du support 10. Sur la figure 10a, l'élément chauffant 33 est disposé sur la face 30b opposée à la face 30a recouverte du matériau métallique réfléchissant. L'élément chauffant 33 peut consister en une résistance chauffante mince fixée sur le support 30, par exemple par collage, par laminage, par dépôt électrochimique, par dépôt électrolytique, par impression, par sérigraphie, par métallisation sous vide, par chauffage, par fixation mécanique, ou par tout autre procédé d'adhésion de couche fine.

Selon l'invention, l'élément chauffant 33 se présente sous la forme d'un circuit électrique flexible dans lequel sont intégrés un ou plusieurs filaments chauffants. Ces derniers se présentent par exemple sous la forme de bandes de métal (cuivre, cuivre-nickel, aluminium, ...), d'une épaisseur de 1 µm à 50 µm, aménagées sur la face 30b du support 30. Ces filaments chauffants peuvent recouvrir le support flexible souple 30 de manière homogène comme représenté à la figure 18a, de sorte que la densité de puissance électrique développée par l'élément résistif chauffant 33 soit identique sur toute la face 30b dudit support.

Dans une variante de réalisation représentée à la figure 18c, les filaments chauffants recouvrent le support 30 de manière non homogène. La densité surfacique de filaments chauffants peut varier selon l'axe longitudinal X"-X" du support flexible 30 de manière à ce que l'on puisse chauffer davantage l'entrée de la cuve de mesure 3 contenant le trou 332 par lequel rentre le fluide d'haleine exhalé. Cette variation peut être régulière ou non (par exemple, sur la figure 18c, il existe trois zones de densités distinctes). Selon l'axe longitudinal X"-X", la densité surfacique de filaments chauffants est donc plus importante du côté de la face 30b contenant le trou 332 que du côté opposé contenant le trou 338. En effet, les perturbations thermiques en dynamique, induites par la circulation du fluide d'haleine lorsqu'un utilisateur souffle dans l'embout 1a vont dans le sens d'un plus grand refroidissement de l'entrée 332 de la cuve 3 par rapport à la sortie 338 de ladite cuve. Un tel agencement de l'élément résistif chauffant 33 permet de compenser ces gradients de température inhérents à l'échantillonnage du fluide d'haleine exhalé et de rendre la température de la cuve 3 la plus homogène possible afin de gagner en précision de mesure.

Dans une autre variante de réalisation représentée à la figure 18d, les filaments chauffants forment deux sous-ensembles résistifs 33' et 33" connectés électriquement en parallèle. Les nœuds N₁ et N₂ relient ces deux sous-ensembles résistifs 33', 33" qui occupent deux zones distinctes du support flexible 30 de surfaces sensiblement identiques. La densité surfacique des filaments chauffants est sensiblement identique sur ces deux zones de sorte que les deux sous-ensembles résistifs 33' et 33" ont avantageusement les mêmes valeurs de résistance en statique (lorsqu'aucun souffle n'est effectué). Ainsi, le chauffage homogène de la cuve 3 pour atteindre la température de consigne est plus aisé qu'avec le support 30 de la figure 18c. En dynamique, le déséquilibre de température induit par l'air expiré entre l'entrée 332 et la sortie 338 de la cuve 3 se traduit par une variation plus importante de la résistance du sous-ensemble résistif 33' (situé du côté du trou d'entrée 332) par rapport à celle du sous-ensemble résistif 33" (situé du côté du trou de sortie 338). La connexion électrique en parallèle de ces deux sous-ensembles résistifs permet de compenser ce déséquilibre en injectant (au niveau de l'un des nœuds N₁ ou N₂) davantage de courant électrique dans le sous-ensemble résistif de plus faible résistance électrique. Cette configuration permet d'auto-réguler le chauffage de la cuve 3 et d'homogénéiser en permanence sa température, à la fois en statique et en dynamique, ce qui n'est pas le cas des variantes des figures 18a et 18c.

L'exemple non limitant de la figure 10d présente uniquement la connexion en parallèle de deux sous-ensembles résistifs, mais peut être étendu à une multitude de sous-ensembles résistifs sans pour autant que la surface occupée par chacun desdits sous-ensemble ne soit forcément identique. Ainsi, il peut être intéressant que la densité surfacique de chacun des sous-ensembles résistifs ne soit pas identique pour équilibrer la valeur de leur résistance électrique en fonction des déséquilibres thermiques identifiés dans la cuve de mesure 3.

L'élément chauffant 33 est relié à des fils conducteurs 330 qui sont intégrés dans une bande 331 du support 30 qui fait saillie de l'âme dudit support. Les fils 330 sont destinés à être connectés à une batterie 7. En pratique, la source de courant est déterminée pour délivrer une tension comprise entre 0,1 Volts et 5 Volts et la puissance développée par l'élément chauffant 33 soit comprise entre 10 mW/cm² et 10 W/cm².

Une régulation en température de l'élément chauffant 33 (et donc de la cuve 3), par exemple autour de 40°C, peut être prévue.

Pour que la loi de BEER LAMBERT s'applique convenablement, la cuve de mesure 3 doit être maintenue à une température constante, et ce malgré d'une part les échauffements externes à la cuve 3 produits par l'ensemble des composants électroniques de l'unité de commande 9 et du moyen de mesure 34, 35 et d'autre part par le refroidissement de l'intérieur de la cuve 3 lors d'un souffle. Il apparaît donc avantageux de pouvoir réguler la température de la cuve 3.

Un contrôle direct de la température de la cuve 3 par un ou plusieurs capteurs de température (du type PT100, thermistance, SAW, ...) placés en son sein aurait pu être envisagé. Toutefois, un capteur de température ne permet d'effectuer qu'une mesure ponctuelle de la température dans la cuve. En pratique, une régulation de la température n'est pas performante avec un seul point de température. Il faut donc prévoir plusieurs capteurs de température répartis le long de la cuve 3. Cette solution est toutefois onéreuse et entraîne une gestion fastidieuse des températures mesurées. En outre, les capteurs de températures sont relativement encombrants, de sorte que leur présence réduit la compacité de l'appareil A.

Pour surmonter ces problèmes techniques, la régulation de la température de la cuve 3 est préférentiellement réalisée sans capteur de température placé en son sein. L'élément résistif chauffant 33, une fois connecté électriquement à l'unité de commande 9, sert donc à la fois à produire l'énergie pour chauffer la cuve de mesure 3 et en même temps à mesurer la température moyenne de cette cuve. Cette solution diffère des solutions connues de l'état de l'art en ce qu'elle mutualise les deux fonctions de chauffage et de mesure de la température de la cuve 3 à partir d'un unique composant, à savoir l'élément résistif chauffant 33.

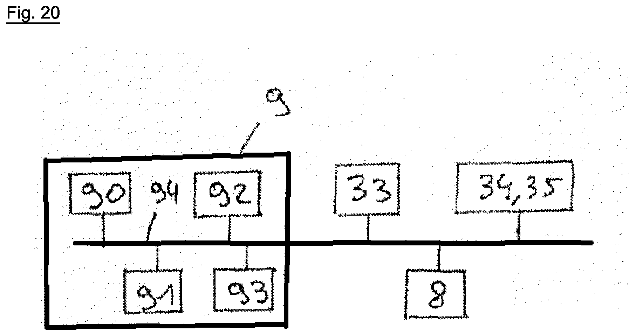

Pour connaître la température moyenne de la cuve 3, on utilise la résistance électrique de l'élément résistif chauffant 33. Lors de la mesure de concentration d'un composant de fluide d'haleine exhalé, la température de la cuve de mesure 3 doit être régulée en permanence autour d'une température cible qui correspond à une résistance de consigne de l'élément résistif chauffant 33. L'unité de commande 9 régule l'énergie électrique injectée dans l'élément résistif chauffant 33 grâce à une boucle de contre-réaction basée sur la mesure en temps réel de la résistance dudit élément et l'objectif d'atteindre la résistance de consigne correspondant à la température de chauffage cible. Plus précisément, l'unité de commande 9 contient un microprocesseur 90 (figure 20) doté d'une chaîne d'acquisition qui réalise la mesure en temps réel de l'intensité et du courant, donc de la résistance, de l'élément résistif chauffant 33. La boucle de rétroaction (ou de contre-réaction) est quant à elle effectuée par un régulateur PID (acronyme de « Proportionnel, Intégral, Dérivé ») ou tout autre moyen de régulation connu de l'homme du métier.

Cette méthode de régulation présente l'avantage d'être beaucoup plus précise et moins onéreuse que celle mettant en œuvre des capteurs de température. En outre, quand un utilisateur souffle dans l'embout 1a, l'entrée de la cuve 3 est refroidie par la circulation du fluide d'haleine. La méthode de régulation employée permet de rééquilibrer très rapidement la température de chauffage entre l'entrée et la sortie de la cuve 3, de sorte qu'on obtient une température homogène dans toute ladite cuve (de l'entrée jusqu'à la sortie).

Le support 30 est conformé sous la forme d'un tube pour former la cuve de mesure 3. Le support 30 est roulé, manuellement ou automatiquement, de manière à former un tube cylindrique. La longueur de la cuve 3 ainsi formée est comprise entre 5 mm et 200 mm, préférentiellement inférieure ou égale à 100 mm. Son diamètre interne est inférieur à 15 mm, par exemple compris entre 4 mm et 15 mm. Dans le cas où la cuve 3 n'est pas de section circulaire, mais de section carrée, rectangulaire, ellipsoïdale ou d'une autre forme polygonale, le support 30 est plié ou roulé de manière à former une cuve 3 ayant cette section particulière. Le support 30 est conformé de sorte que le matériau métallique réfléchissant forme la surface interne de la cuve 3. Cette disposition permet d'optimiser les longueurs des trajets optiques dans la cuve 3, tout en conservant une quantité de lumière suffisante jusqu'au récepteur décrit plus avant dans la description. Il en résulte que la cuve de mesure 3 peut être relativement courte.

Lorsque la cuve 3 est mise en forme, le support 30 a naturellement tendance à se dérouler (ou se déplier) pour retrouver sa forme plate d'origine. Pour remédier à cela, le support 30 comporte deux bordures longitudinales 32a, 32b opposées, qui sont solidarisées l'une à l'autre par collage ou soudage pour maintenir la conformation dudit support sous forme de tube. Les deux extrémités longitudinales des bordures 32a, 32b peuvent être mises bord-à-bord et solidarisées par collage, soudage, etc. Selon un mode préféré de réalisation, une des bordures 32a présente une bande 320 libre, représentée à la figure 18b, dépourvue d'élément résistif chauffant 33. Cette bande 320 est continue et s'étend dans toute la longueur du support 30. Sa largeur est par exemple comprise entre 2 mm et 5 mm. La bande 320 sert de zone d'encollage. Lorsque le support 30 est conformé sous forme de tube, la bande 320 vient en recouvrement de la bordure opposée 32b. Au niveau de cette zone de recouvrement, l'épaisseur de la cuve 3 est donc double. Toutefois, étant donné que la bande 320 est dépourvue d'élément résistif chauffant 33, on ne double pas l'élément résistif chauffant 33 au niveau de cette zone de recouvrement.

Dans cette configuration, la densité surfacique des filaments constitutifs de l'élément résistif chauffant 33 peuvent avantageusement varier selon l'axe transversal Y"-Y" orthogonal à l'axe X"-X". Ainsi, ladite densité est différente au centre de la face 30b de l'article 3 et sur les bordures (au niveau de la bordure 32b et au niveau de la bordure intérieure de la bande libre 320 qui est opposée à la bordure 32a). On obtient donc un chauffage homogène de la cuve 3, sans zone de surchauffe ou de dissipation thermique liée à la zone de recouvrement.

Dans une variante de réalisation, les deux bordures longitudinales 32a, 32b du support 3 ne sont pas solidarisées l'une à l'autre. Le support 3 est conformé en tube puis glissé dans un autre tube de préférence non conducteur métallique et/ou thermique, par exemple un tube fin en polyimide (par ex : Kapton^{®}), polyépoxyde, polyester, résine époxy renforcée de fibre de verre, substrat d'aluminium (par ex : support COOL-CLAD^{®} commercialisé par la société AI TECHNOLOGY), etc.

Selon encore une autre variante de réalisation, les deux bordures longitudinales 32a, 32b du support 3 ne sont pas solidarisées l'une à l'autre. Le support 3 est conformé en tube, sans solidarisation des bordures longitudinales 32a, 32b. Le boîtier 2 est formé d'un carter monobloc, comportant un aménagement définissant un logement dans lequel est glissé le support 3 ainsi conformé en tube.

La cuve de mesure 3 et/ou sa méthode de régulation de température, peuvent bien évidemment être employés dans d'autres appareils de mesure. Ces modes de réalisations ne sont toutefois pas couverts par la présente invention revendiquée.

Lorsque la cuve 3 est conformée sous forme de tube, celui-ci est ouvert à ses deux extrémités. Le moyen de mesure dépend du gaz à analyser. On utilise préférentiellement un moyen de mesure photométrique qui détermine la concentration du gaz. En se rapportant aux figures 4, 5, et 6, un émetteur de rayonnement infrarouge 34 est monté au niveau d'une extrémité 3a de la cuve 3 de manière à ce que ladite cuve soit traversée par un rayonnement infrarouge. L'émetteur 34 émet avantageusement dans des longueurs d'ondes comprises entre 2 µm et 15 µm. L'émetteur 34 est avantageusement couplé à un réflecteur optique en cône (non représenté) permettant d'augmenter l'intensité du rayonnement infrarouge émis et de concentrer ce rayonnement dans la cuve 3. Un détecteur de rayonnement infrarouge 35 est monté à l'extrémité opposée 3b. L'émetteur 34 et le détecteur 35 sont du type connu de l'homme du métier. L'émetteur 34 peut par exemple consister en un MEMS et le détecteur 35 en un détecteur pyro-électrique.

Lorsque le fluide d'haleine circule dans la cuve 3 entre les deux extrémités 3a, 3b, ledit fluide est susceptible de refroidir l'émetteur 34 et/ou le détecteur 35, lequel refroidissement risque de perturber les mesures. Pour remédier à cela, on interpose préférentiellement une chambre ou fenêtre étanche devant l'émetteur 34 et une autre chambre ou fenêtre étanche devant le détecteur 35. Ces chambres ou fenêtres sont étanches au fluide d'haleine de sorte que ledit fluide ne puisse pas rentrer au contact de l'émetteur 34 et du détecteur 35. Avantageusement, les chambres ou fenêtres sont chacune formée par un anneau 36 qui s'emmanche dans l'extrémité respective 3a, 3b de la cuve 3 et à l'intérieur de laquelle est inséré une lentille ou disque transparent, par exemple réalisé en verre ou en tout autre matériau dont la transparence est maximale aux longueurs d'ondes infrarouges utiles à la mesure de concentration du composant d'intérêt du gaz d'haleine exhalé.

Dans une variante de réalisation non représentée, on inverse les places de l'émetteur 34 et du détecteur 35, de sorte que l'émetteur 34 soit situé en entrée de cuve, et le détecteur 35 soit en sortie de cuve, près du trou 338.

Selon une caractéristique de l'invention, la cuve 3, le moyen de mesure 34, 35, le moyen de pompage 8 et l'unité de commande 9 sont réunis sur un support commun 4a, de manière à former un ensemble unitaire préhensible. Cet ensemble unitaire est installé de manière amovible dans le logement du boîtier 2. Ces différents moyens peuvent ainsi être facilement assemblés sur le support commun 4a amovible, à l'extérieur du boîtier 2, sur établi par exemple. L'opérateur peut donc mettre en place les composants, les fixer et les connecter dans un espace de travail bien plus accessible que celui défini par le corps du boîtier 2. Le montage des différents moyens est de fait plus rapide que dans l'art antérieur précité. L'opérateur n'a plus qu'à insérer l'ensemble unitaire ainsi formé dans le boîtier 2 pour finaliser la fabrication de l'appareil A. De même, en cas panne d'un des composants électroniques, l'opérateur a simplement à retirer l'ensemble unitaire défaillant du boîtier 2, et à le remplacer par un autre ensemble unitaire en bon état de marche. La durée de neutralisation de l'appareil A est donc considérablement réduite. L'ensemble unitaire défaillant pourra être ultérieurement expertisé et réparé, sans impacter l'utilisation de l'appareil A.

Sur les figures 4, 5 et 6, le support commun 4a se présente sous la forme d'une pièce allongée ayant un axe longitudinal X'-X' (qui coïncide également avec l'axe longitudinal de la cuve 3). Le support commun 4a est réalisé dans un matériau rigide, par exemple en plastique (ex : PVC, ABS, PC), en carbone, en matériau composite, etc. Il peut être obtenu par moulage, par extrusion ou par tout autre procédé convenant à l'homme du métier. Il peut nécessiter un traitement de surface particulier, notamment un traitement anti-inflammabilité.

Sur la figure 5, le support commun 4a est formé par l'assemblage de deux pièces 4A, 4B présentant un plan de joint parallèle à l'axe X'-X'. Ces pièces 4A, 4B sont maintenues entre-elles après assemblage, par une fixation mécanique (vissage, clipsage...), une fixation thermique (soudage...) ou une fixation chimique (collage, ...).

Les pièces 4A, 4B présentent chacune un aménagement complémentaire en forme de berceau 430 ou demi-tube qui, lorsque lesdites pièces sont assemblées, forment un logement tubulaire 43 dans lequel est installée la cuve 3. Ce logement tubulaire 43 présente une ouverture 431 sous forme de fente par laquelle la bande 331 du support 30 sort dudit logement.

D'autres logements 44 et 45 sont aménagés aux extrémités du logement 43, pour recevoir respectivement l'émetteur 34 (et sa fenêtre étanche 36) et le détecteur 35 (et sa fenêtre étanche 36). Selon le type de moyen de mesure utilisé, un seul logement dédié à ce dernier peut être prévu.

Un support commun 4a en deux parties 4A, 4B facilite le montage de la cuve 3 et de l'émetteur 34 et détecteur 35. La cuve 3 conformée sous forme de tube est posée dans le berceau 430 de la pièce inférieure 4A. On positionne ensuite l'émetteur 34 dans le berceau formant le logement 44 et le détecteur 35 dans le berceau formant le logement 45. Les chambres ou fenêtres étanches 36 peuvent être assemblées préalablement aux extrémités 3a, 3b de la cuve 3 ou mises en position pendant l'installation de l'émetteur 34 et du détecteur 35. Lorsque ces composants 3, 34, 35, 36, sont installés dans la pièce inférieure 4A, on assemble la pièce supérieure 4B pour les maintenir en position.

Un montage des composants 3, 34, 35, 36 est également possible en utilisant un support commun 4a monobloc pourvu du logement tubulaire 43 aux extrémités duquel sont aménagés les autres logements tubulaires 44 et 45. Le montage se fait alors en insérant la cuve 3 dans le logement 43, puis en plaçant les chambres ou fenêtres 36 à chacune des extrémités de ladite cuve, et enfin en installant l'émetteur 34 et le détecteur 35.

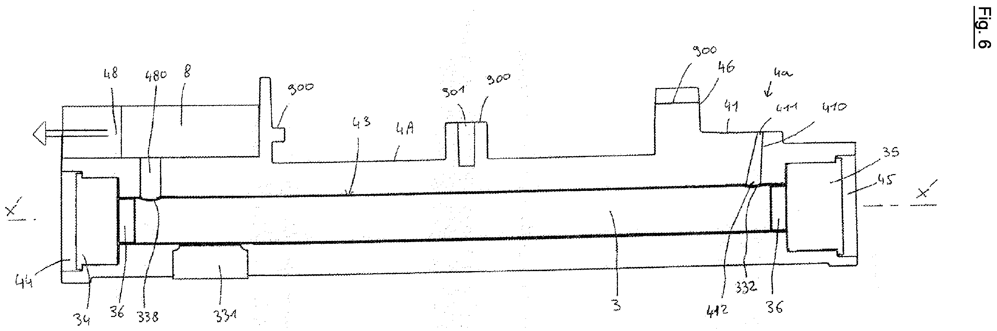

En se rapportant aux figures 6 et 9, le support commun 4a comporte un perçage 410 débouchant dans le logement 43 dans lequel est installée la cuve 3. Plus particulièrement, le perçage 410 présente un premier orifice 411 qui débouche sur une paroi extérieure 41 du support commun 4a et un second orifice 412 qui débouche dans le logement 43, au droit d'un trou 332 (figures 18a et 18b) aménagé dans le support 30 de la cuve 3. Le perçage 410 est ainsi en communication fluidique avec la cuve 3. Le perçage 410 peut avoir une section constante. Le perçage 410 est toutefois préférentiellement conique, avec un évasement du cône vers la cuve 3, c'est-à-dire que le diamètre du premier orifice 411 est inférieur au diamètre du second orifice 412. Cette configuration permet de réduire fortement la vitesse du fluide d'haleine à l'entrée de la cuve 3. En réduisant cette vitesse, on limite les problèmes aérauliques et on réduit les turbulences dans la cuve 3 (lesquelles turbulences sont susceptibles de ralentir le remplissage de ladite cuve et de diminuer la précision de la mesure de concentration du composant d'intérêt du gaz d'haleine exhalé. Un perçage 410 en cône permet ainsi une répartition plus homogène du fluide dans la cuve 3. Des résultats satisfaisants sont également obtenus avec un perçage 410 cylindrique, mais avec de moins bonnes performances en termes de rapidité de remplissage et d'homogénéisation de la répartition du fluide dans la cuve 3.

En se rapportant plus particulièrement à la figure 9, le support commun 4a comporte un autre perçage 420 débouchant dans une chambre 42 aménagée dans ledit support. Cette chambre 42 est davantage visible sur la figure 4a. Un capteur de pression (non représenté) est installé dans la chambre 42. Ce capteur de pression est du type connu de l'homme du métier et est connecté à l'unité de commande 9 décrite plus avant dans la description. Le perçage 420 présente un premier orifice 421 qui débouche sur la paroi 41 du support commun 4a et un second orifice 422 qui débouche dans la chambre 42. La paroi 41 porte donc les deux orifices 411 et 421.

Comme illustré sur la figure 9, lorsque l'embout 1a est installé dans l'ouverture 21A, l'orifice de sortie 112a débouche au-dessus de la paroi 41 du support commun 4a de sorte qu'il est en communication fluidique avec le premier perçage 410 et avec le second perçage 420. L'âme 11 de l'embout 1a, la paroi interne du tube 2A et la paroi 41 du support commun 4a peuvent s'agencer ensemble et coopérer pour délimiter une chambre dans laquelle sont situés les orifices 112a, 411 et 421. L'échantillon du fluide d'haleine exhalé qui sort de l'embout 1a par l'orifice de sortie 112a pénètre ainsi dans le perçage 410 pour rentrer dans la cuve de mesure 3 et pénètre dans le perçage 411 pour rentrer dans la chambre 42. La mesure de la pression dans la chambre 42 est utilisée par l'unité de commande 9 pour déduire la valeur du débit du fluide d'haleine circulant dans la cuve de mesure 3.

Le support commun 4a comporte également un logement 48 dans lequel est installé le moyen de pompage 8. Ce dernier se présente sous la forme d'un ventilateur plat installé dans le logement 48. Ce dernier comporte un perçage 480 débouchant dans le logement 43 dans lequel est installée la cuve 3 de sorte que les logements soient en communication fluidique. Plus particulièrement, le perçage 480 présente un premier orifice qui débouche dans le logement 43, au droit d'un trou 338 (figures 10a et 10b) aménagé dans le support 30 de la cuve 3. Le perçage 480 présente également un second orifice qui débouche dans le logement 48, au droit du ventilateur 8.

Sur les figures 18a et 18b, les trous 332 et 338 sont respectivement situés au niveau d'un bord latéral du support 30. Leurs dimensions sont ajustées aux diamètres des perçages 410 et 480.

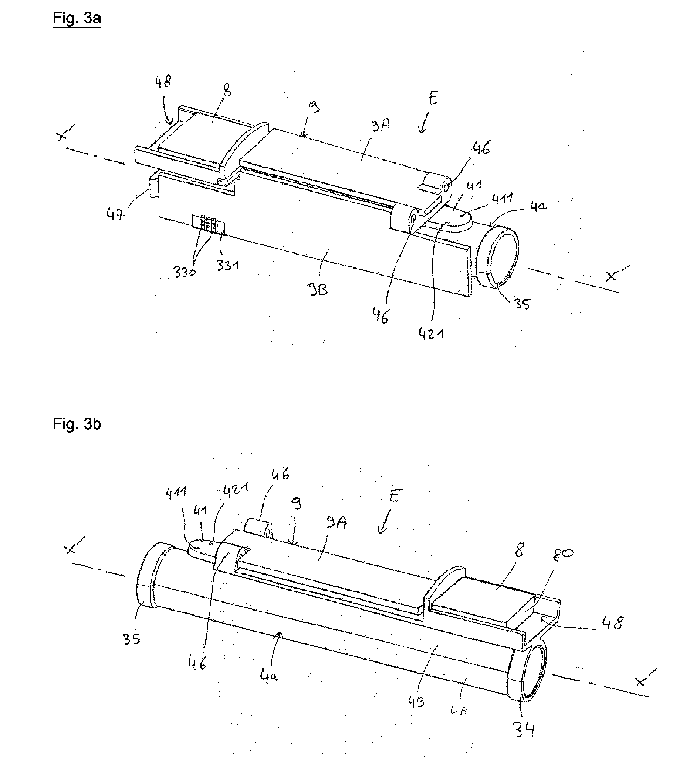

Comme schématisé sur la figure 3b, le ventilateur 8 comporte une sortie 80 par laquelle est expulsé le fluide d'haleine. Cette sortie 80 est configurée pour que la direction d'expulsion du fluide d'haleine (illustrée par la double flèche sur les figures 6a et 6b), soit parallèle à l'axe longitudinal X'-X' de la cuve 3. Le fluide d'haleine est ainsi extrait dans le boîtier 2, parallèlement à l'axe longitudinal X-X de ce dernier. Ce fluide d'haleine ainsi extrait circule tout le long du boîtier 2 jusqu'à sortir par l'ouverture 220B du bouchon 22B, éventuellement en refroidissant sur son passage les autres éléments incorporés dans ledit boîtier. En outre, la demanderesse a constaté que cette direction d'expulsion évite tout retour intempestif du fluide d'haleine dans la cuve 3.

Sur les figures 3a et 3b, l'unité de commande 9 se présente sous la forme d'une ou plusieurs cartes de circuit imprimé 9A, 9B portant des composants électroniques (non représentés) permettant de contrôler et piloter l'appareil A et notamment le moyen de mesure 34, 35, et le moyen de pompage 8. L'unité de commande 9 est également adaptée pour activer et contrôler le chauffage de la cuve 3 et le capteur de pression précité.

### Deuxième mode de réalisation.

Selon un deuxième mode de réalisation, les deux chambres sont réalisées dans le support commun 4b, comme illustré sur les figures 10, 11 et 14. Cette configuration permet notamment de simplifier davantage la forme de l'embout 1b sans affecter la précision de la mesure de concentration du gaz d'haleine effectuée au sein de la cuve de mesure 3.

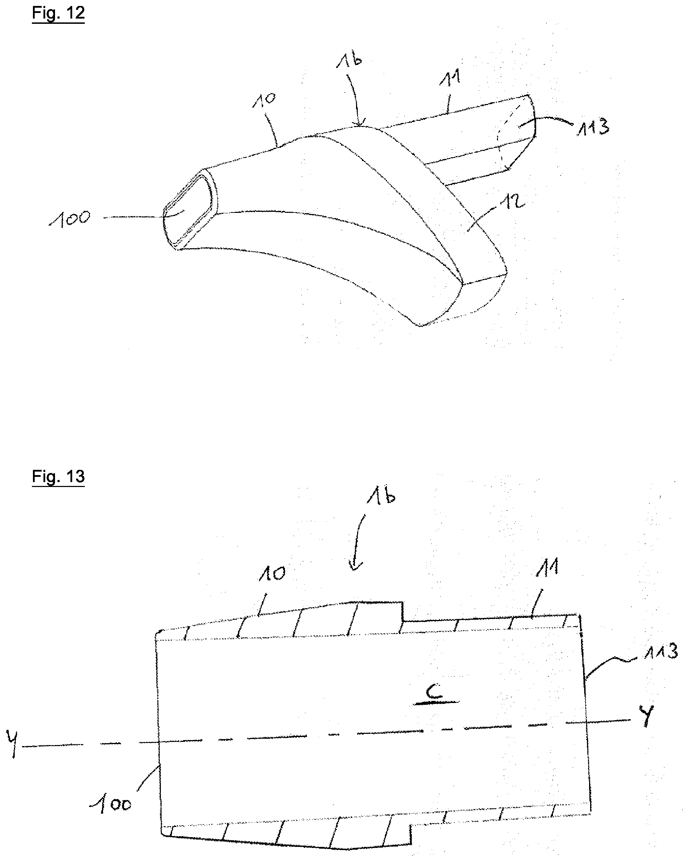

Sur les figures 12 et 13, l'embout 1b comporte une chambre C munie d'un orifice d'entrée 100 et d'un orifice de sortie 113. Les sections de ces deux orifices sont identiques ou sensiblement identiques. Comparativement à l'embout 1a du premier mode de réalisation, cette configuration permet de réduire le niveau de complexité, et par conséquent le nombre d'étapes de fabrication, de l'embout 1b. Il permet également de réduire les quantités de matière utilisées pour fabriquer cette pièce et donc d'abaisser son coût de revient. En revanche, la taille de l'orifice de sortie 113 étant sensiblement plus grand que celle de l'orifice de sortie 112 de l'embout 1a, l'embout 1b envoie une plus grande fraction de fluide d'haleine dans l'appareil A. Or le fluide d'haleine est susceptible de contenir des particules qui peuvent encrasser ledit appareil. Aussi, dans la configuration des figures 12 et 13, il peut être avantageux de munir l'embout 1b d'un filtre (non représenté) en amont ou au niveau de son orifice de sortie 113, de manière à réduire sensiblement l'encrassement de l'appareil A. Le terme « *amont*» fait référence à la direction du flux du fluide d'haleine exhalé.

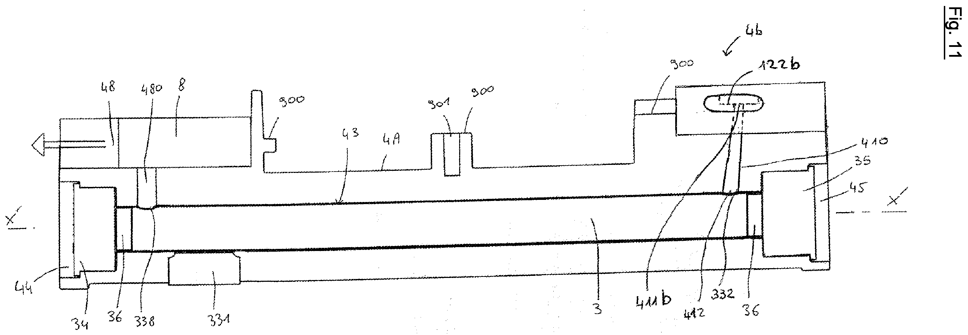

Cet embout 1b peut être associé au support commun 4b illustré sur les figures 10 et 11. La première chambre 101b et la seconde chambre 110b sont réalisées sur le support commun 4b, en amont de la cuve 3. La seconde chambre 110b est disposée à l'intérieur de la première chambre 101b.

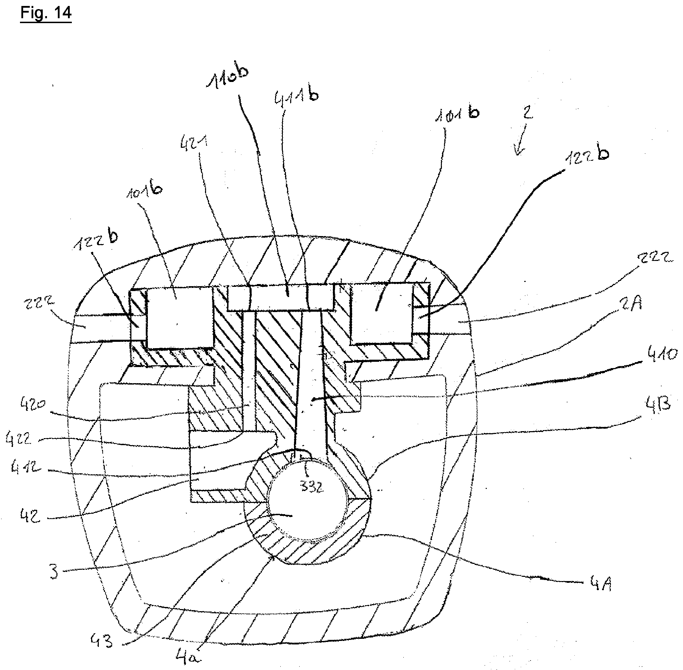

La seconde chambre 110b a des dimensions réduites par rapport à la première chambre 101b, comme dans le premier mode de réalisation. Les deux chambres 101b et 110b sont ouvertes dans leur partie supérieure. Comme illustré sur la figure 14, ces ouvertures sont refermées et étanchéifiées lors de l'assemblage du support commun 4b avec le boîtier 2A. La première chambre 101b comporte un orifice d'entrée 100b au travers duquel le fluide d'haleine exhalé pénètre dans ladite première chambre. Cet orifice d'entrée 100b est adjacent à l'orifice de sortie 113 de l'embout 1b, et communique avec la chambre C, lorsque ledit embout est installé dans le boîtier 2. La seconde chambre 110b comporte également un orifice d'entrée 111b débouchant dans la première chambre 101b.

Comme pour le premier mode de réalisation, le support commun 4b comporte un premier perçage 410 débouchant dans la cuve 3 et un second perçage 420 débouchant dans la chambre 42.

Le perçage 410 présente un premier orifice 411b qui débouche dans la seconde chambre 110b et un second orifice 412 qui débouche dans le logement 43, au droit du trou 332. Le perçage 410 met ainsi en communication fluidique la seconde chambre 110b avec la cuve 3. L'orifice 411b est l'orifice de sortie au travers duquel passe la partie du fluide d'haleine exhalé circulant dans la seconde chambre 110b (équivalent de l'orifice 112 de l'embout 1a du premier mode de réalisation).

Le perçage 420 présente un premier orifice 421 qui débouche dans la seconde chambre 110b et un second orifice 422 qui débouche dans la chambre 42.

Les parois latérales externes de la première chambre 101b comportent les orifices de sortie 122b au travers desquels la fraction du fluide d'haleine exhalé qui ne circule pas dans la cuve de mesure 3 est expulsé à l'air ambiant. Pour les mêmes raisons que celles évoquées précédemment en référence au premier mode de réalisation, ces orifices de sortie 122b sont des orifices latéraux, orientés selon une direction Z-Z qui est perpendiculaire à l'alignement Y-Y des orifices 100, 113, 100b, et 110b. En se rapportant à la figure 14, lorsque le support 4b est installé dans le tube 2A, les orifices de sortie 122b sont en vis-à-vis des orifices 222 disposés latéralement sur le boîtier 2.

Egalement pour les mêmes raisons que celles évoquées précédemment en référence au premier mode de réalisation, et comme illustré sur la figure 10, l'entrée 111b de la seconde chambre 110b est avantageusement située en amont des orifices de sortie 122b. Et préférentiellement, les parois latérales de la seconde chambre 110b ont des dimensions selon l'axe Y-Y supérieures à celles des orifices de sortie 122b.

Lorsque l'embout 1b est installé dans l'ouverture 21A du boîtier 2, le premier perçage 410, le second perçage 420 et les orifices de sortie 122b sont en communication fluidique avec l'orifice de sortie 113 de l'embout 1b. La distance qui sépare alors l'orifice d'entrée 100 de l'orifice d'entrée 411 est typiquement inférieure à 50 mm. Cette configuration présente l'avantage de ne plus nécessiter un alignement précis entre l'embout 1b et le support commun 4b, ce qui abaisse les tolérances de fabrication et donc le coût des embouts. Par contre, elle nécessite toujours un alignement de précision entre le support commun 4b et le tube 2A afin que les orifices de sortie 122b soient alignés avec les orifices latéraux 222.

### Troisième mode de réalisation.

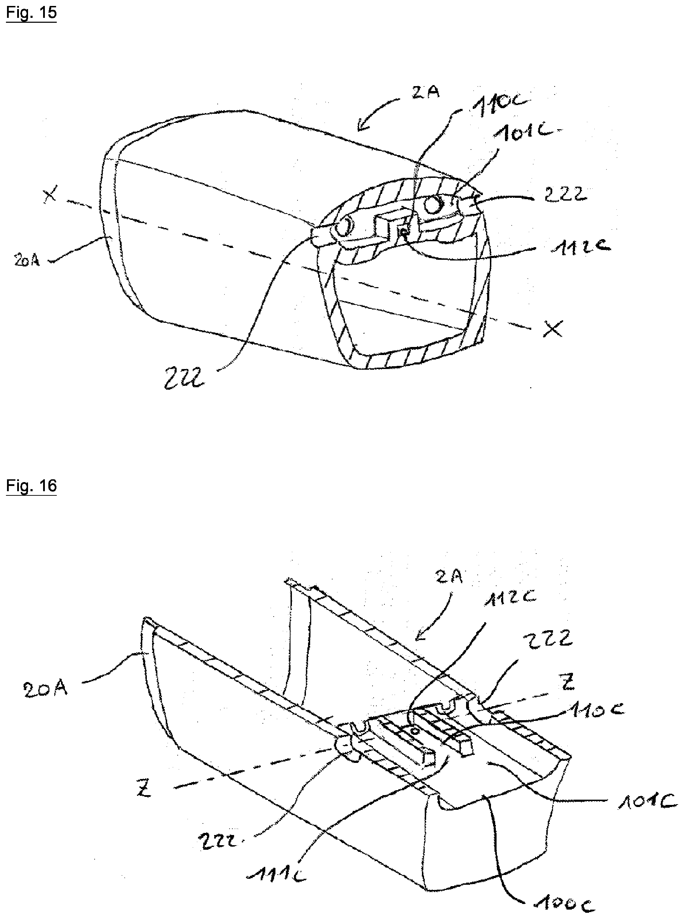

Selon un troisième mode de réalisation, les deux chambres sont réalisées dans le boîtier 2, comme illustré sur les figures 15 à 17. Cette configuration permet de simplifier la conception dans la mesure où l'embout 1b du deuxième mode de réalisation peut être associé au support commun 4a du premier mode de réalisation, sans affecter la précision de la mesure de concentration du gaz d'haleine effectuée au sein de la cuve de mesure 3.

La première chambre 101c et la seconde chambre 110c sont réalisées dans le boîtier 2 et plus particulièrement dans le tube 2A, en amont de la cuve 3. La seconde chambre 110c est disposée à l'intérieur de la première chambre 101c.

La seconde chambre 110c a des dimensions réduites par rapport à la première chambre 101c, comme dans le premier mode et le deuxième mode de réalisation. La première chambre 101c comporte un orifice d'entrée 100c au travers duquel le fluide d'haleine exhalé pénètre dans ladite première chambre. Cet orifice d'entrée 100c est adjacent à l'orifice de sortie 113 de l'embout 1b, et communique avec la chambre C, lorsque ledit embout est installé dans le boîtier 2. La seconde chambre 110c comporte également un orifice d'entrée 111c débouchant dans la première chambre 101c.

La partie du fluide d'haleine exhalé circulant dans la seconde chambre 110c est extraite par un orifice de sortie 112c qui est en communication fluidique avec la cuve de mesure 3 comme expliqué plus en avant dans la description.

L'autre partie du fluide d'haleine exhalé qui ne circule pas dans la seconde chambre 110a, est expulsée à l'air ambiant par les orifices de sortie 222 réalisés dans les parois latérales du tube 2A et qui débouchent directement dans la première chambre 101c.

Pour les mêmes raisons que celles évoquées précédemment en référence au premier mode de réalisation :
- les orifices de sortie 222 sont des orifices latéraux, orientés selon une direction Z-Z qui est perpendiculaire à l'alignement Y-Y des orifices 100, 113, 100c, et 110c ;
- l'orifice d'entrée 111c de la seconde chambre 110c est avantageusement située en amont des orifices de sortie 222. Et préférentiellement, les parois latérales de la seconde chambre 110c ont des dimensions selon l'axe X'-X' supérieures à celles des orifices de sortie 222.

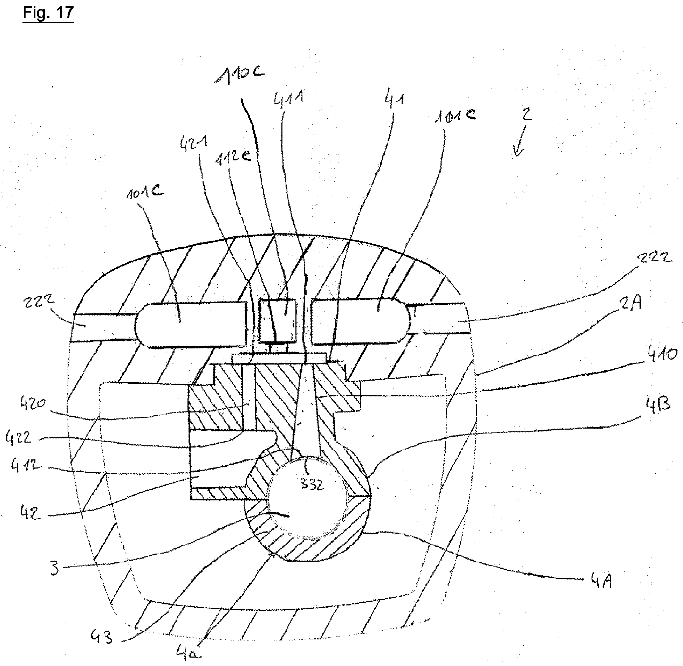

Comme illustré sur la figure 17, lorsque l'embout 1b est installé dans l'ouverture 21A, l'orifice de sortie 112c débouche au-dessus de la paroi 41 du support commun 4a de sorte qu'il est en communication fluidique avec le premier perçage 410 et avec le second perçage 420. La paroi interne du tube 2A et la paroi 41 du support commun 4a peuvent s'agencer ensemble et coopérer pour délimiter une chambre étanche dans laquelle sont situés les orifices 112c, 411 et 421. L'échantillon du fluide d'haleine exhalé qui sort de la seconde chambre 110c par l'orifice de sortie 112c pénètre ainsi dans le perçage 410 pour rentrer dans la cuve de mesure 3 et pénètre dans le perçage 411 pour rentrer dans la chambre 42.

La distance qui sépare alors l'orifice d'entrée 100 de l'orifice de sortie 112c est typiquement inférieur à 50 mm. Là encore, cette configuration présente l'avantage de ne plus nécessiter d'alignement précis entre l'embout 1b, le support commun 4a et le tube avant 2A du boîtier 2, ce qui abaisse les tolérances de fabrication et donc le coût de l'ensemble de ces pièces. De plus, un alignement de l'axe longitudinal de la seconde chambre 110c avec l'axe d'insertion de l'embout 1b permet au fluide d'haleine expiré d'avoir un trajet direct entre l'entrée 100 de l'embout 1b et la sortie 112c du tube avant 2A.

Quel que soit le mode de réalisation (premier, deuxième ou troisième), pour adapter le confort de l'utilisateur lorsqu'il souffle dans l'appareil A et/ou pour faire varier le volume du fluide d'haleine échantillonné dans la cuve de mesure 3, il est possible de faire varier le ratio R₁ entre la somme S_{OSE} des sections moyennes des orifices de sortie 122a, 122, 222 et la plus petite des sections moyennes de la seconde chambre 110a, 100b, 110c Ssc ou de l'orifice de sortie 112a, 411b, 112c Sos, tel que R₁ = S_{OSE} / min(Ssc ; Sos). Ce ratio R₁ est avantageusement compris entre 5 et 50 (5 < R₁ < 50) de sorte que 2% à 20% du fluide d'haleine passant au travers de l'orifice d'entrée 100 ressorte par l'orifice de sortie 112. En d'autres termes, 80% à 98% du fluide d'haleine exhalé dans la première chambre 101a, 101b, 101c est expulsé à l'air ambiant par l'orifice de sortie 122a, 122b, 222. Avantageusement, la dimension des différents orifices ou chambres sont adaptés de façon à ce que le ratio R₁ soit compris entre 12 et 35.

En se rapportant à la figure 20, l'unité de commande 9 comprend notamment un ou plusieurs processeurs ou microprocesseurs 90, une ou plusieurs mémoires 91, un module de communication 92, éventuellement une interface réseau 93, qui sont mutuellement connectés via un bus 94. Une ou plusieurs applications informatiques - ou programmes informatiques - sont enregistrées dans la ou les mémoires 91 et dont les instructions (ou codes), lorsqu'elles sont exécutées par le ou les processeurs 90 permettent de réaliser les fonctionnalités de l'appareil A. Par souci de clarté, il faut comprendre au sens de l'invention que *« l'appareil A fait quelque chose* » signifie « *l'application informatique exécutée par le processeur de l'appareil A fait quelque chose* »*.* Tout comme « *l'application informatique fait quelque chose* » signifie « *l'application informatique exécutée par le processeur de l'appareil A fait quelque chose* »*.*

La ou les mémoires 91 doivent être considérées comme un dispositif de stockage également adapté pour stocker des données et/ou des fichiers de données telles que des précédentes mesures. Il peut s'agir d'une mémoire native ou d'une mémoire rapportée telle qu'une carte Secure Digital (SD).

Le module de communication 92 est adapté pour échanger des signaux radiofréquences transmis sans fil avec un terminal mobile appairé avec l'appareil A. Dans le but de simplifier la conception, les signaux radiofréquences sont préférentiellement des signaux utilisant un protocole Bluetooth. Toutefois, d'autres protocoles tels que : ISM, Wifi, ANT, ZIGBEE..., peuvent être utilisés. Le terminal mobile se présente sous la forme d'un Smartphone (téléphone intelligent) du type iPhone^{®}, Samsung Galaxy^{®}, ou sous la forme d'un autre terminal électronique, par exemple une tablette électronique tactile (du type iPad^{®}, Samsung GalaxyTab^{®}, fonctionnant avec un système d'exploitation de type Windows, Mac, iOS, Android, etc. Ce terminal mobile est adapté pour être exploité par un utilisateur, qui est en pratique, le propriétaire de l'appareil A.

L'interface réseau 93 est adaptée pour établir une communication entre l'appareil A et un serveur informatique distant. L'interface réseau 93 peut par exemple comprendre un module GSM fournissant une connectivité de réseau internet à l'appareil A. De manière générale, l'interface réseau 93 a pour fonction de gérer les connexions entre l'appareil A et un réseau internet.

Le moyen de mesure 34, 35, le moyen de pompage 8, l'élément résistif chauffant 33 de la cuve 3 et le capteur de pression installé dans la chambre 42 peuvent être connectés au bus commun 94.

Sur les figures annexées, le support commun 4a, 4b comporte un ou plusieurs aménagements pour recevoir l'unité de commande 9, et plus particulièrement les cartes de circuit imprimé 9A, 9B. Ces aménagements se présentent sous la forme de bordures et/ou plots 900 sur lesquelles reposent les cartes 9A, 9B. Ces aménagements 900 sont préférentiellement répartis sur plusieurs côtés du support commun 4a, 4b de sorte que plusieurs cartes 9A, 9B peuvent être fixées sur ledit support selon différentes orientations. On obtient alors un ensemble unitaire E particulièrement compact. La fixation des cartes 9A, 9B sur le support commun 4a, 4b se fait au moyen de vis qui s'engagent dans des taraudages 901 réalisés dans des plots 900 (figures 4 et 5).

Pour simplifier la conception, une des cartes 9B supporte préférentiellement le capteur de pression précité. Une fois installé sur le support commun 4a, 4b, cette carte 9B vient recouvrir une face ouverte de la chambre 42 de sorte que ladite face ouverte soit obturée de manière étanche au fluide d'haleine. Ce recouvrement est réalisé de manière à ce que le capteur de pression soit logé dans la chambre 42.

La cuve de mesure 3, le moyen de mesure 34, 35, le moyen de pompage 8 et l'unité de commande 9 sont donc réunis sur le support commun 4a, 4b de manière à former l'ensemble unitaire E. Cet ensemble E est préhensible, en ce sens qu'il peut être facilement manipulé par un opérateur.

En se rapportant aux figures 2a et 2b, l'ensemble unitaire E est installé de manière amovible dans le logement du boîtier 2, et plus particulièrement à l'intérieur du tube avant 2A. Cette installation est réalisée très simplement en glissant l'ensemble E dans le tube 2A, selon l'axe X-X. L'ensemble E est avantageusement maintenu en position dans le tube 2A au moyen de vis 26 qui sont en prise avec une paroi dudit tube et qui s'engagent dans des taraudages 46 aménagés sur le support commun 4a (figures 2a, 3a, 4, 6).

L'autre tube 2B est adapté pour recevoir une batterie électrique 7 adaptée pour alimenter l'appareil A et plus particulièrement : le moyen d'information 21, le moyen de mesure 34, 35, le moyen de pompage 8, l'unité de commande 9. La batterie 7 est également adaptée pour alimenter l'élément résistif chauffant 33 dans le cas où celui-ci est intégré dans le support 30 de la cuve 3 et utilisé. La batterie 7 se présente par exemple sous la forme d'un assemblage d'une ou plusieurs piles capables de délivrer entre 2 Volts et 24 Volts. La batterie 7 peut également se présenter sous la forme d'une batterie rechargeable du type de celles utilisées dans des Smartphones, auquel cas, le boîtier 2 est pourvu d'une connectique adaptée pour brancher l'appareil A sur secteur afin de recharger ladite batterie.

La batterie 7 est avantageusement dimensionnée pour assurer au moins 75 cycles de mesures pour une température ambiante comprise entre 0°C et 50°C. Le choix des composants (moyen de mesure 34, 35, moyen de pompage 8, élément chauffant 33, unité de commande 9, moyen d'informations 21) et leur mode de gestion durant un cycle de mesure permettent de minimiser la puissance électrique nécessaire à chaque cycle de mesure. En particulier, la faible épaisseur de la couche de matériau métallique réfléchissant de la cuve de mesure 3 permet de limiter l'inertie thermique de l'ensemble et donc de chauffer très rapidement ladite cuve. La puissance électrique nécessaire au chauffage de cette dernière est donc optimisée.

En se rapportant aux figures 2a et 3a, la batterie 7 est connectée à une lame flexible conductrice 70 qui est adaptée pour venir en contact avec une lame conductrice 47 aménagée sur le support commun 4a, 4b et plus particulièrement connectée à la carte 9B. Le contact entre les lames 70 et 47 est réalisé lors de l'assemblage des tubes 2A, 2B, lequel contact autorise une alimentation électrique de l'ensemble unitaire E.

Le tube 2B est également adapté pour recevoir le moyen d'information 21 et le bouton marche/arrêt 20. A cet effet, le tube 2B présente une ouverture 221B aménagée sur sa paroi, laquelle ouverture est configurée pour recevoir un support 221 sur lequel sont fixés le bouton 20 et l'écran 21.

Le fonctionnement de l'appareil A va maintenant être décrit avec plus de détail.

L'utilisateur appuie sur le bouton 20 de manière à mettre en fonctionnement l'appareil A.

Il souffle dans l'embout 1a, 1b au travers de l'ouverture 100. Le flux du fluide d'haleine pénètre dans la première chambre 101a, 101b, 101c. La majeure partie du fluide d'haleine est évacué à l'air ambiant par les orifices de sortie latéraux 122a, 122b, 222.

Un échantillon du fluide d'haleine pénètre dans la seconde chambre 110a, 110b, 110c ressort de ladite deuxième chambre, sous pression, par l'orifice de sortie 112a, 411b, 112c.

Cet échantillon de fluide d'haleine pénètre dans la cuve de mesure 3 au travers du passage 410. Il est à noter que c'est la pression de souffle du fluide d'haleine dans l'embout 1a, 1b qui force la circulation de l'échantillon dans la cuve et non pas une éventuelle dépression créée dans ladite cuve par le moyen de pompage 8. Le débit du fluide d'haleine qui circule dans la cuve 3 est donc susceptible de varier selon l'utilisateur. Aussi, il est intéressant de mesurer dans la chambre 42 la pression du fluide d'haleine circulant dans la cuve 3 pour calculer le débit dudit fluide.

La concentration d'un ou plusieurs composants du gaz de fluide d'haleine circulant dans la cuve 3 est mesuré par le moyen de mesure 34, 35.

Le fluide d'haleine ressort de la cuve de mesure 3 par le perçage 480, le moyen de pompage 8 servant à son extraction. Ce dernier est davantage utilisé pour compenser les pertes de charges et purger la cuve 3, que pour créer une dépression dans ladite cuve servant à l'échantillonnage du fluide d'haleine. Le fluide d'haleine traverse le boîtier 2 pour être rejeté à l'air ambiant au travers de l'ouverture 220B.

La concentration de gaz mesurée par le moyen de mesure 34, 35 est traitée par l'unité de commande 9 en fonction de la pression mesurée dans la chambre 42, et donc du débit, de manière à calculer la concentration du composant dans le gaz de fluide d'haleine (par exemple : la masse du composant par litre de gaz expiré). La valeur de la concentration ainsi calculée peut alors être affichée sur l'écran 21.

On peut également piloter l'appareil A depuis un terminal mobile (Smartphone, tablette, ...) appairé audit appareil. Les communications entre l'appareil A et le terminal se font par l'intermédiaire du module de communication 92 précité. L'utilisateur peut être amené à installer une ou plusieurs applications informatiques dans son terminal mobile pour mettre en œuvre tout ou partie de l'invention depuis ledit terminal, et notamment la procédure d'appairage. Ces applications informatiques peuvent être préinstallées sur le terminal mobile. L'utilisateur a toutefois la possibilité de rechercher ces applications informatiques sur une boutique en ligne telles que Google Play^{®}, Itunes^{®} ou sur un site internet dédié, et ensuite les télécharger sur son terminal mobile.

Par souci de clarté, il faut comprendre au sens de l'invention que *« le terminal mobile fait quelque chose »* signifie « *l'application informatique exécutée par un processeur du terminal mobile fait quelque chose* »*.* Tout comme « *l'application informatique fait quelque chose »* signifie « *l'application informatique exécutée par un processeur du terminal mobile fait quelque chose* »*.*

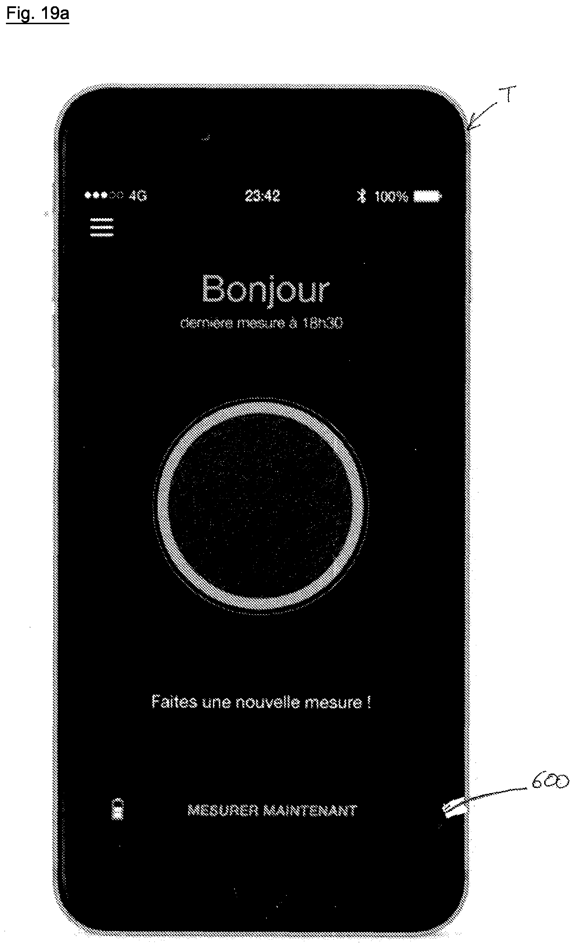

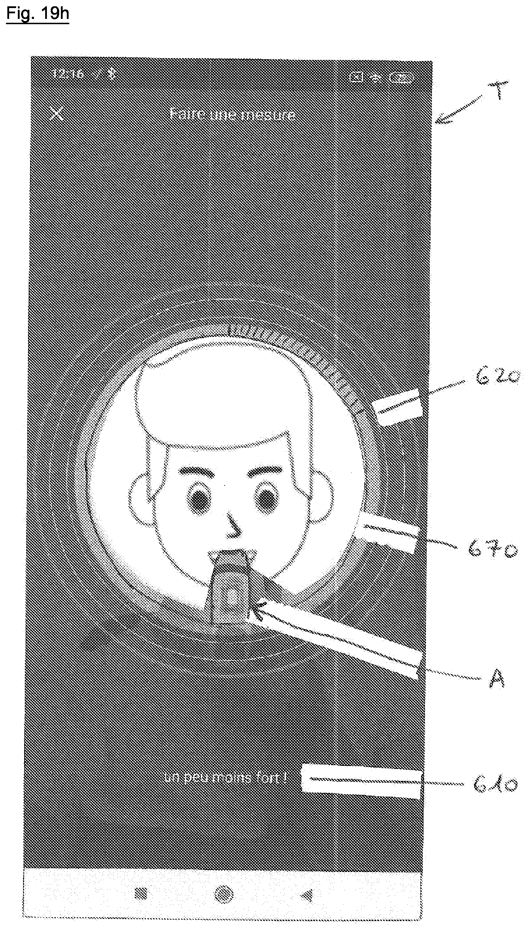

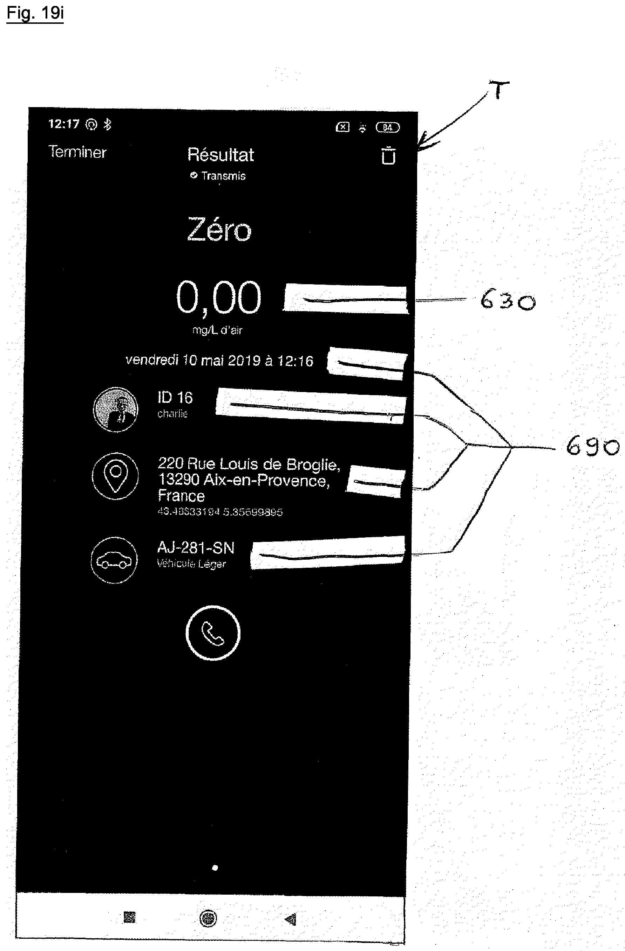

Lorsque cette application informatique est lancée depuis le terminal mobile, plusieurs interfaces graphiques sont affichées sur un écran dudit terminal de manière à contrôler, guider et/ou informer l'utilisateur. Ces interfaces graphiques sont illustrées sur les figures 19a à 19i. Elles s'appliquent à une utilisation de l'appareil A comme éthylomètre ou éthylotest.

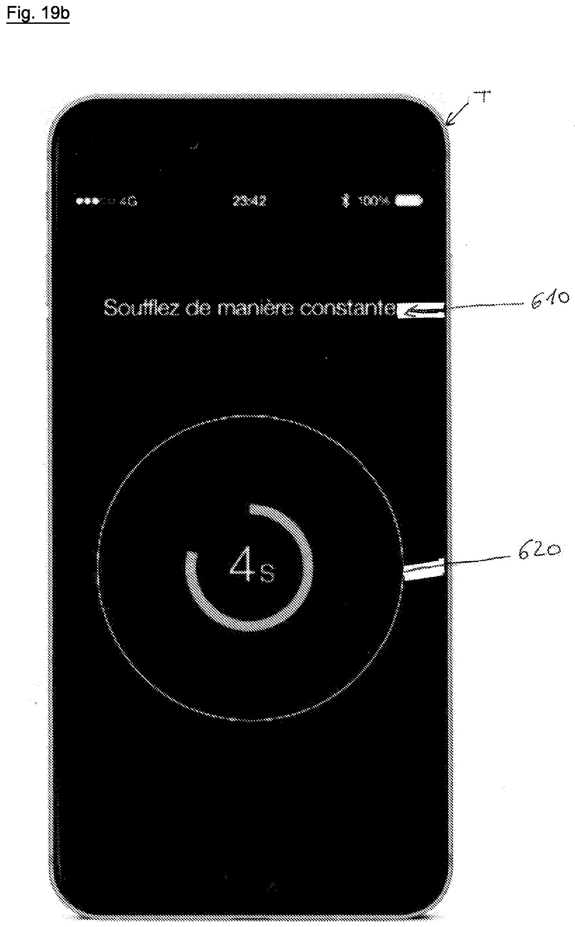

Figure 19a : l'application informatique affiche une touche sélectionnable 600 sur l'écran du terminal T, laquelle touche permet de lancer une mesure. La sélection de la touche 600 peut être optionnelle. En effet, le terminal T peut communiquer en continu avec l'appareil A, et dès qu'un souffle est détecté par le capteur de pression installé dans la chambre 42, ledit terminal affiche automatiquement l'écran de la figure 19b.

Figure 19b : lorsque l'utilisateur actionne la touche 12, le terminal T affiche des instructions 610 pour opérer la mesure. Cette instruction 610, par exemple du type : « *soufflez de manière constante* » ou « *un peu moins fort !*», peut varier tout au long du souffle pour guider l'utilisateur, et ce afin que le volume d'air expiré soit conforme à l'échantillon d'air attendu par l'appareil A pour que la mesure soit valide. Un chronomètre 620 s'affiche également sur l'écran du terminal T pour décompter le temps pendant lequel l'utilisateur doit souffler dans l'embout 1. L'affichage du décompte se fait préférentiellement par le graphisme, mais peut également s'afficher en toutes lettres (ex : 4 secondes).

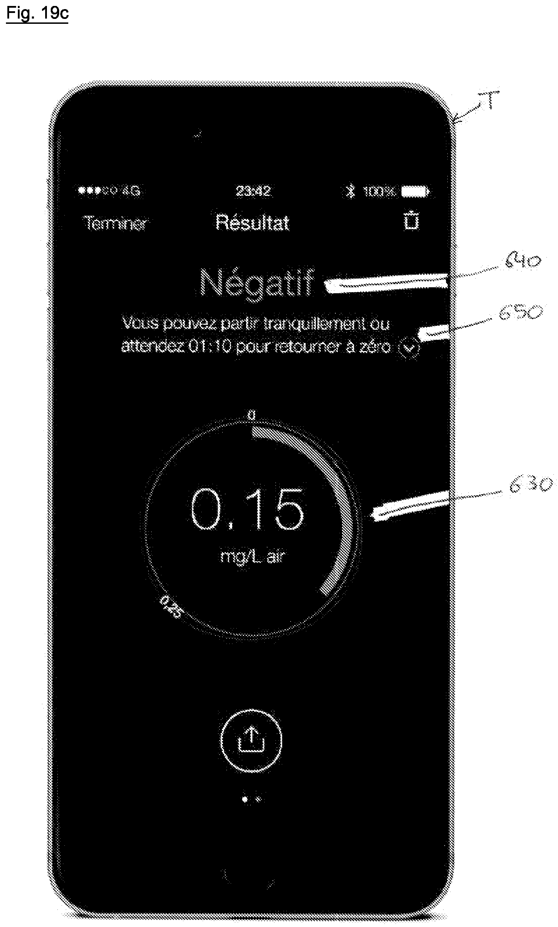

Figure 19c : le terminal mobile affiche la concentration mesurée 630 par l'appareil A (ex : alcoolémie). Le terminal T peut comparer la valeur de cette concentration avec des valeurs seuils. Par exemple, en France, la limite autorisée en matière d'alcoolémie est de 0,25 mg/Lₐᵢᵣ (données 2018, hors jeunes conducteurs et transports de personnes). En deçà de ce seuil, un utilisateur peut conduire un véhicule motorisé terrestre (moto, voiture). Au-delà de ce seuil, l'utilisateur n'est pas autorisé à conduire son véhicule et encourt des sanctions en cas de non-respect de cette interdiction :
- une amende et un retrait de points sur son permis de conduire si la concentration d'alcool est comprise entre 0,25 mg/Lₐᵢᵣ et 0,40 mg/Lₐᵢᵣ.
- une amende, une suspension ou une annulation du permis de conduire, voire une peine de prison, si la concentration d'alcool est supérieure ou égale à 0,40 mg/Lₐᵢᵣ.

Les valeurs seuils sont paramétrables dans un menu de l'application non représenté. Ils dépendent de la catégorie de conducteur (expérimenté, débutant ou professionnel) mais également de la législation de chaque pays. Si l'utilisateur accepte sa géolocalisation, alors l'application informatique peut lui proposer, lorsqu'il change de pays, de mettre à jour automatiquement les valeurs seuils correspondant à sa catégorie dans ce nouveau pays.

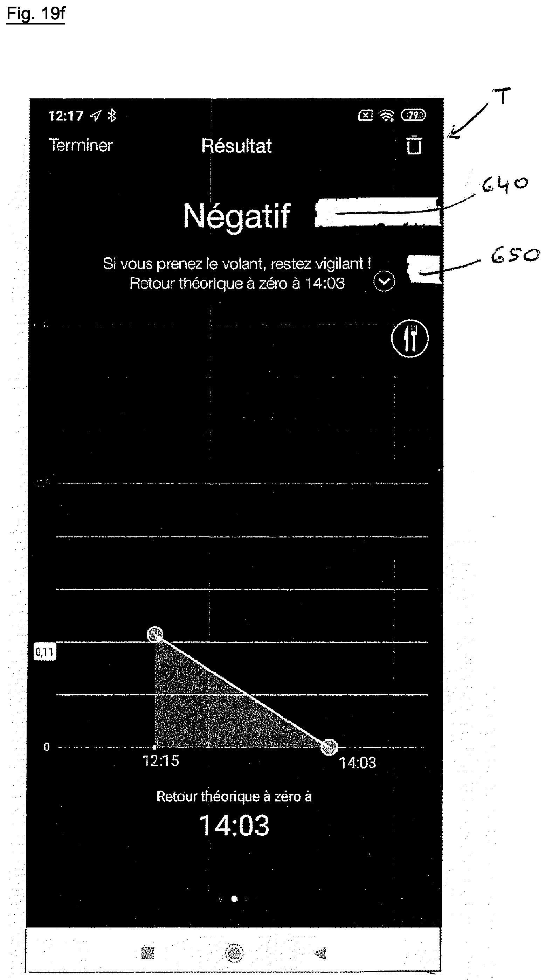

Sur la figure 19c, l'alcoolémie mesurée 630 est de 0,15 mg/Lₐᵢᵣ. Cette concentration étant inférieure au seuil légal autorisé, le terminal T peut afficher le résultat du test 640, par exemple « *NEGATIF*» ou « *NON REPREHENSIBLE* ». Le terminal T peut en outre afficher des recommandations et/ou d'autres informations 650. Par exemple le terminal peut afficher un message indiquant à l'utilisateur qu'il peut utiliser son véhicule (ex : *vous pouvez partir tranquillement* ») ou que sa dernière mesure n'est plus valide. Le terminal T peut également calculer le temps nécessaire à l'utilisateur pour que son alcoolémie retombe à 0 mg/Lₐᵢᵣ et afficher sur le terminal T ce temps sous forme de message (ex : « *retour à zéro estimé à 1 heure et 10 minutes* ») et/ou d'une courbe comme représentée à la figure 19f. Ce temps peut notamment être calculé en fonction de données morphologiques de l'utilisateur préalablement renseignées (ex : sexe, âge, taille, poids) et/ou selon que l'utilisateur a ingéré ou pas des aliments au moment de sa prise d'alcool. Les temps de redescente de l'alcoolémie sont plus longs si la personne est à jeun et inversement. Une autre possibilité est de proposer à l'utilisateur, à chaque mesure d'une concentration d'alcool détectée, de rentrer des renseignements sur les circonstances de son ingestion (heure de la prise d'alcool, type de boisson, heure du dernier repas...), ces données étant enregistrées par l'application informatique. Par un procédé d'apprentissage, l'application informatique pourrait alors prédire de plus en plus précisément, pour un utilisateur donné et au fur et à mesure de ses souffles, le temps pour que son alcoolémie retombe à 0 mg/Lₐᵢᵣ.

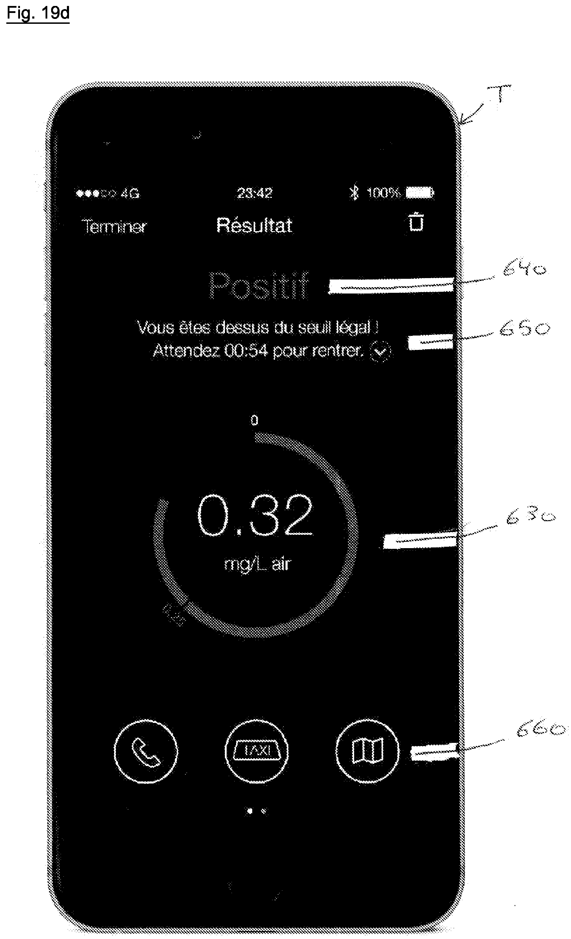

Figure 19d : l'alcoolémie mesurée 630 est ici égale à 0,32 mg/Lₐᵢᵣ. Cette concentration est comprise entre les seuils 0,25 mg/Lₐᵢᵣ et 0,40 mg/Lₐᵢᵣ. Le terminal T affiche le résultat du test 640, par exemple : « *POSITIF* » ou *« REPREHENSIBLE ».* Le terminal T affiche également des recommandations et/ou d'autres informations 650 appropriées pour ce cas. Par exemple le terminal affiche un message indiquant à l'utilisateur qu'il ne peut pas utiliser son véhicule (ex : *« vous êtes au-dessus du seuil légal»* et/ou *« ne prenez pas votre véhicule* »)*.* Le terminal T affiche également le temps nécessaire à l'utilisateur pour que son alcoolémie passe en dessous du seuil légal autorisé (ex : *« Retour au seuil légal estimé dans 54 minutes*»)*.* Ce temps est calculé en fonction des données morphologiques de l'utilisateur préalablement renseignées et/ou selon que l'utilisateur a ingéré ou pas des aliments au moment de sa prise d'alcool. Les temps de redescente de l'alcoolémie sont plus longs si la personne est à jeun et inversement. L'application informatique peut également proposer des services à l'utilisateur qui est au-delà du seuil autorisé pour la conduite. Ces services se matérialisent sur le terminal T par des icônes sélectionnables 660 permettant de contacter, par exemple, un service de taxi.

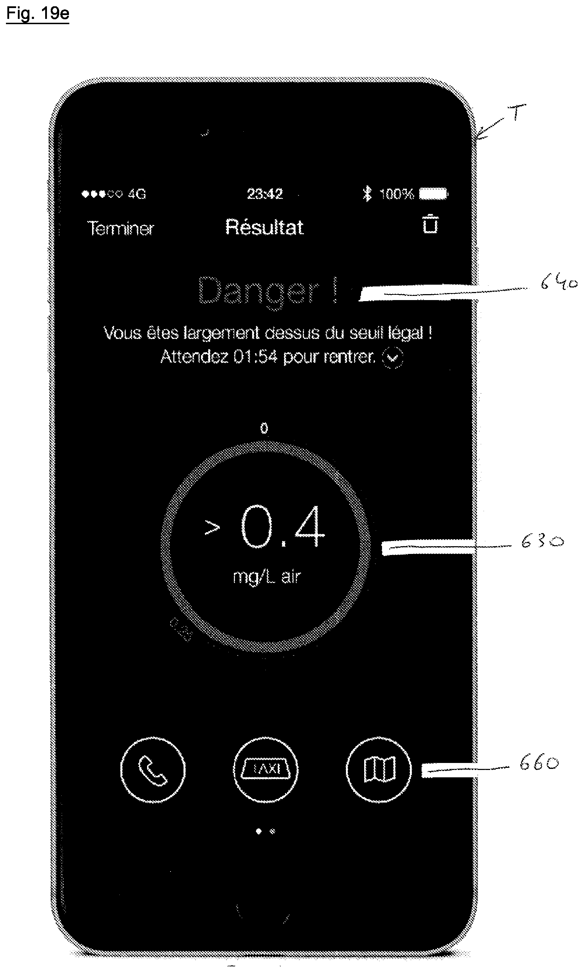

Figure 19e : l'alcoolémie mesurée 630 est ici égale à 0,4 mg/Lₐᵢᵣ. Cette concentration place l'utilisateur dans un contexte des plus risqués. Le terminal T affiche le résultat du test 640, par exemple : *« DELICTUEL* » et/ou *« DANGER ».* Le terminal T affiche également des recommandations et/ou d'autres informations 650 appropriées pour ce cas. Par exemple le terminal affiche un message recommandant fortement à l'utilisateur de ne pas utiliser son véhicule (ex : « *vous êtes largement au-dessus du seuil légal»* et/ou *« ne prenez surtout pas le volant* »)*.* Le terminal T affiche également le temps nécessaire à l'utilisateur pour que son alcoolémie passe en dessous du seuil légal autorisé (ex : *« attendez 1 heure et 54 minutes pour rentrer*»)*.* Ce temps est calculé en fonction des données morphologiques de l'utilisateur préalablement renseignées et/ou selon que l'utilisateur a ingéré ou pas des aliments au moment de sa prise d'alcool. Les temps de redescente de l'alcoolémie sont plus longs si la personne est à jeun et inversement.

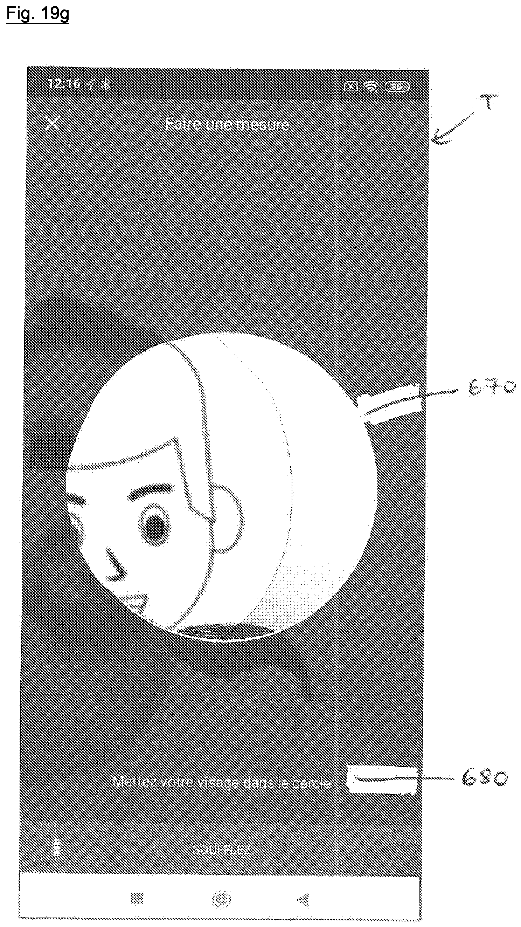

Dans une variante de réalisation, on enregistre et on associe, dans une base de données, un moyen d'identification de l'appareil A et un moyen d'identification de l'utilisateur. Préalablement à la mesure, on acquière, depuis le terminal T de l'utilisateur, le moyen d'identification de l'appareil A et le moyen d'identification de l'utilisateur. On analyse le moyen d'identification de l'appareil A acquis et le moyen d'identification de l'utilisateur acquis. Et seulement en cas de correspondance entre le moyen d'identification de l'appareil (A) acquis et le moyen d'identification de l'utilisateur acquis, on transmet à l'unité de commande 9 une instruction pour opérer la mesure, laquelle instruction est générée depuis le terminal mobile T. Cette variante est notamment illustrée par la figure 19g.

Figure 19g : lorsque l'utilisateur actionne la touche 600, l'application informatique affiche sur l'écran du terminal T une zone de reconnaissance 670 (représentée ici sous la forme d'un cercle) et des préconisations 680 (par exemple du type : « *mettez votre visage dans le cercle* ») pour le placement adéquat du visage de l'utilisateur et de l'appareil A dans ladite zone de reconnaissance 670. L'acquisition en temps réel des images de l'utilisateur et de l'appareil se font via une caméra du terminal T. Ces images sont traitées par un algorithme propre à l'application et notamment un algorithme de reconnaissance faciale. L'image du visage de l'utilisateur vu de face, qui correspond en réalité à un ensemble unique de coordonnées propres à la physionomie de l'utilisateur, est préalablement enregistrée dans une base de données lors de la configuration de l'application afin qu'elle puisse être comparée et reconnue lors du procédé d'identification de l'utilisateur. Plus précisément, ces données sont enregistrées dans un compte personnel de l'utilisateur sur un serveur sécurisé, l'application informatique pouvant accéder à ces données lorsque l'utilisateur renseigne son nom de compte et son mot de passe. En ce qui concerne l'appareil A, sa forme et ses éventuels marquages sont également connus de l'application, et un algorithme de reconnaissance de la forme et/ou de marquage doit permettre de l'identifier sous différentes faces et à différents angles afin que sa reconnaissance soit indépendante de la façon dont l'utilisateur souffle dans ledit appareil.

D'autres moyens d'identification de l'utilisateur (par exemple par reconnaissance d'empreintes digitales ou par reconnaissance d'iris) peuvent également être envisagés. De même, on peut identifier par d'autre moyen l'appareil A, par exemple par lecture d'un QR-Code installé sur ledit appareil.

Figure 12i : Le terminal T peut également afficher in fine un rapport qui contient l'alcoolémie mesurée 630 ainsi qu'un ensemble d'informations 690 propres à l'utilisateur (identifiant, immatriculation du véhicule) et à la mesure (date et heure, coordonnées de géolocalisation). Ce rapport peut être stocké dans le compte personnel de l'utilisateur sur un serveur sécurisé, ou directement envoyé à une tierce personne (par exemple, à un responsable d'entreprise qui a le devoir de veiller à la sobriété de ses employés, même s'ils sont à distance).

L'utilisation d'éthylotests comme dispositifs anti-démarrage de véhicules ou comme dispositifs de contrôle pour les entreprises disposant de flottes de véhicules ou de machines à risque permettent d'empêcher la conduite de véhicules ou l'utilisation d'outils dangereux aux personnes dont le taux d'alcoolémie est supérieur aux seuils fixés par la loi ou par les règlements internes aux entreprises. Or il existe des moyens de contourner les règles pour les utilisateurs ayant dépassé les seuils autorisés, par exemple en utilisant des systèmes de pompes permettant d'injecter de l'air ou en faisant souffler des tierces personnes sobres dans lesdits éthylotests.

Le procédé de vérification de l'identité de l'utilisateur proposé ici vise à remédier à de tels agissements en fiabilisant les mesures d'alcoolémie lorsque l'utilisateur est contraint d'opérer une telle mesure, mais libre de ne pas souffler par lui-même dans le dispositif de contrôle. Il est avantageux en ce qu'il contourne de possibles utilisations frauduleuses des éthylotests, ce qui représente un danger pour la conduite, tout en informant l'utilisateur du temps d'attente nécessaire pour qu'il repasse en deçà du seuil de concentration d'alcool autorisé.

L'agencement des différents éléments et/ou moyens et/ou étapes de l'invention, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes, sans s'écarter de la portée de l'invention établie par les revendications.

En particulier :
- L'appareil A n'est pas nécessairement de forme allongée. Il peut rester compact dans une configuration où par exemple l'ensemble unitaire E et la batterie 7 sont disposés l'un à côté de l'autre.
- Le boîtier 2 peut être monobloc, c'est-à-dire formé d'un seul tube allongé dont la paroi interne définit le logement. Il peut également être formé de plus de deux tubes 2A, 2B, par exemple trois ou quatre tubes emboîtés ensemble.
- Le moyen d'information 21 et/ou le bouton marche/arrêt 20 peuvent être installés sur le tube 2A.
- Le moyen de mesure peut être adapté pour traiter des signaux autres que des signaux infrarouges et/ou mettre en œuvre une technique d'analyse autre que la photométrie.
- On peut forcer la circulation de l'échantillon dans la cuve 3 par la dépression créée dans ladite cuve par le moyen de pompage 8.
- La sortie 80 du ventilateur 8 peut être configurée pour que la direction d'expulsion du fluide d'haleine, soit normale à l'axe longitudinal X'-X' de la cuve 3, ou incliné par rapport à cet axe. L'ouverture 220B peut alors être positionnée n'importe où dans le boîtier, notamment sur une paroi du tube 2A ou du tube 2B.
- Les caractéristiques relatives au support commun 4a, à la cuve de mesure 3, à la régulation en température, décrites en référence au premier mode de réalisation, s'appliquent également au deuxième mode et troisième mode de réalisation.
- Sur les figures annexées, la seconde chambre 110a, 110b, 110c est disposée à l'intérieur de la première chambre 101a, 101b, 101c. La seconde chambre 110a, 110b, 110c peut toutefois être dans une position adjacente à la première chambre 101a, 101b, 101c, les deux chambres étant dans le même alignement ou disposées côte-à-côte par exemple.
- La seconde chambre 110a, 110b, 110c n'a pas nécessairement des dimensions réduites par rapport à celles de la première chambre 101a, 101b, 101c. Elle peut en effet avoir les mêmes dimensions ou avoir des dimensions supérieures. Il est utile de purger la seconde chambre avant d'effectuer une mesure de manière à ce que cette dernière soit la plus précise possible. Aussi, plus le volume de la seconde chambre 110a, 110b, 110c est important, plus son temps de purge sera élevé. Il est donc avantageux d'avoir une seconde chambre dont les dimensions sont réduites pour la purger la plus rapidement possible et obtenir une mesure rapide et précise.

## Revendications

1. Appareil portable de mesure de la concentration d'au moins un composant dans un gaz exhalé par un fluide d'haleine, comportant:
- un embout (1a) au travers duquel le fluide d'haleine est exhalé,
- un boîtier (2) intégrant :
∘ une cuve de mesure (3),
∘ un moyen de mesure (34, 35) adapté à la mesure de la concentration d'au moins un composant dans un gaz du fluide d'haleine circulant dans la cuve de mesure (3),
∘ une ouverture (21A) dans laquelle est installé l'embout (1a),
- une première chambre (101a, 101b, 101c) située en amont de la cuve de mesure (3) et comportant un orifice d'entrée (100, 100b, 100c) au travers duquel le fluide d'haleine exhalé pénètre dans ladite première chambre,
- une seconde chambre (110a, 110b, 110c) située en amont de la cuve de mesure (3), laquelle seconde chambre comporte :
∘ un orifice d'entrée (111a, 111b, 111c) débouchant dans la première chambre (101a, 101b, 101c),
∘ un orifice de sortie (112a, 111b, 112c) en communication fluidique avec la cuve de mesure (3) et au travers duquel passe une partie du fluide d'haleine exhalé,
- un orifice de sortie (122a, 122b, 222) au travers duquel une partie du fluide d'haleine exhalé est expulsé,
appareil dans lequel:
- la seconde chambre (110a, 110b, 110c) est disposée à l'intérieur de la première chambre (101a, 101b, 101c) ou dans une position adjacente à ladite première chambre,
- l'orifice de sortie (122a, 122b, 222) débouche dans la première chambre (101a, 101b, 101c) de sorte que seule une partie du fluide d'haleine exhalé circulant dans ladite première chambre pénètre dans la seconde chambre (110a, 110b, 110c) par l'orifice d'entrée (111a, 111b, 111c) de ladite seconde chambre, l'autre partie du fluide d'haleine exhalé étant expulsée,
appareil **caractérisé en ce que** :
- l'orifice de sortie (122a, 122b, 222) est un orifice de sortie à l'air ambiant,
- la cuve de mesure (3) est réalisée à partir d'un support flexible souple (30) conformé sous la forme d'un tube,
- le support flexible souple (30) comporte une première face (30a) et une seconde face (30b), lesquelles faces sont opposées,
- la première face (30a) est recouverte d'un matériau métallique réfléchissant formant une couche de réflexion optique,
- le support flexible souple (30) intègre un élément résistif chauffant (33), lequel élément chauffant se présente sous la forme d'un circuit électrique flexible dans lequel sont intégrés un ou plusieurs filaments chauffants se présentant sous la forme de bandes de métal d'une épaisseur de 1 µm à 50 µm aménagées sur la seconde face (30b).

2. Appareil selon la revendication 1, dans lequel:
- la première chambre (101a) et la seconde chambre (110a) sont réalisées dans l'embout (1a),
ou
- la première chambre (101c) et la seconde chambre (110c) sont réalisées dans le boîtier (2),
ou
- la cuve de mesure (3), le moyen de mesure (34, 35), un moyen de pompage (8) adapté pour extraire le fluide d'haleine circulant dans la cuve de mesure et l'unité de commande (9) sont réunis sur un support commun (4b) de manière à former un ensemble unitaire préhensible (E), lequel ensemble est installé de manière amovible dans un logement du boîtier (2), la première chambre (101b) et la seconde chambre (110b) étant réalisées dans ledit support commun (4b).

3. Appareil selon l'une des revendications précédentes, comportant au moins l'une des caractéristiques suivantes:
- la seconde chambre (110a, 110b, 110c) a des dimensions réduites par rapport à celles de la première chambre (101a, 101b, 101c).
- l'orifice de sortie (122a, 122b, 222) à l'air ambiant est dimensionné de sorte que 80% à 98% du fluide d'haleine exhalé dans la première chambre (101a, 101b, 101c) soit expulsé à l'air ambiant.

4. Appareil selon l'une des revendications précédentes, dans lequel:
- la cuve de mesure (3), le moyen de mesure (34, 35), un moyen de pompage (8) et l'unité de commande (9) sont réunis sur un support commun (4a, 4b) de manière à former un ensemble unitaire préhensible (E), lequel ensemble est installé de manière amovible dans un logement du boîtier (2),
- le boîtier (2) est formé d'au moins deux tubes allongés (2A, 2B) présentant un axe longitudinal commun (X-X), lesquels tubes s'emboîtent selon ledit axe longitudinal pour définir le logement,
- l'ensemble unitaire (E) est installé dans un des tubes (2A), ce tube (2A) formant un porte-embout dans lequel s'emboîte l'embout (1a, 1b).
- l'autre tube (2B) formant le boîtier (2) est adapté pour recevoir une batterie électrique adaptée pour alimenter l'ensemble unitaire préhensible (E).

5. Appareil selon la revendication 4, comportant au moins une des caractéristiques suivantes:
- le support commun (4a, 4b) est adapté pour assurer une communication fluidique entre la cuve de mesure (3) et l'orifice de sortie (112a, 411 b, 112c) de la seconde chambre (110a, 110b, 110c),
- le support commun (4a, 4b) comporte :
- un logement (43) dans lequel est installée la cuve de mesure (3),
- au moins un logement (44, 45) dans lequel est installé le moyen de mesure (34, 35),
- un logement (48) dans lequel est installé le moyen de pompage (8),
- un ou plusieurs aménagements (900) adaptés pour recevoir l'unité de commande (9).

6. Appareil selon l'une des revendications précédentes, dans lequel:
- la cuve de mesure (3), le moyen de mesure (34, 35), un moyen de pompage (8) et l'unité de commande (9) sont réunis sur un support commun (4a, 4b) de manière à former un ensemble unitaire préhensible (E), lequel ensemble est installé de manière amovible dans le logement du boîtier (2),
- le support commun (4a, 4b) comporte :
∘ un premier perçage (410) qui débouche dans un logement (43) dans lequel est installée la cuve de mesure (3) de sorte que ledit perçage soit en communication fluidique avec ladite cuve,
∘ un second perçage (420) qui débouche dans une chambre (42) aménagée dans ledit support et dans laquelle est installé un capteur de pression,
- l'orifice de sortie (112a, 411 b, 112c) de la seconde chambre (110a, 110b, 110c) est en communication fluidique avec le premier perçage (410) et avec le second perçage (420)
- le premier perçage (410) est conique, lequel premier perçage comporte un premier orifice (411) et un second orifice (412) qui débouche dans le logement (43) dans lequel est installée la cuve de mesure (3), le diamètre dudit premier orifice (411) étant inférieur au diamètre dudit second orifice (412).

7. Appareil selon l'une des revendications précédentes, dans lequel :
- l'orifice d'entrée (100, 100b, 100c) de la première chambre (101 a, 101 b, 101c), l'orifice d'entrée (111 a, 111 b, 111c) de la seconde chambre (110a, 110b, 110c), et l'orifice de sortie (112a, 411b, 112c) de ladite seconde chambre, sont disposés dans le même alignement (Y-Y),
- l'orifice de sortie (122a, 112b, 222) à l'air ambiant est orienté selon une direction (Z-Z) qui est perpendiculaire à cet alignement (Y-Y).

8. Appareil selon l'une des revendications précédentes prise en combinaison avec la revendication 5, dans lequel le logement (48) dans lequel est installé le moyen de pompage (8) comporte un perçage (480) débouchant dans le logement (43) dans lequel est installée la cuve de mesure (3) de sorte que lesdits logements (43, 48) soient en communication fluidique.

9. Appareil selon l'une des revendications précédentes, dans lequel :
- la cuve de mesure (3) et le boîtier (2) présentent chacun un axe longitudinal (X-X ; X'- X'), lesquels axes sont parallèles,
- un moyen de pompage (8) est configuré pour expulser le fluide d'haleine circulant dans la cuve de mesure (3) selon une direction parallèle auxdits axes longitudinaux (X-X ; X'-X').

10. Appareil selon l'une des revendications précédentes, dans lequel :
- le support flexible souple (30) comporte deux bordures longitudinales (32a, 32b) opposées qui sont solidarisées l'une à l'autre par collage pour maintenir la conformation dudit support sous forme de tube,
∘ une desdites bordures (32a) présente une bande dépourvue (320) d'élément résistif chauffant (33).

11. Appareil selon l'une des revendications précédentes, dans lequel :
- le ou les filaments chauffants recouvrent le support flexible souple (30) de manière homogène de sorte que la densité de puissance électrique développée par l'élément résistif chauffant (33) est identique sur toute la seconde face (30b) dudit support (30), ou
- le ou les filaments chauffants recouvrent le support flexible souple (30) de manière non homogène de sorte que la densité de puissance électrique développée par l'élément résistif chauffant (33) varie selon un axe longitudinal (X"-X") et/ou selon un axe transversal (Y"-Y") du support flexible souple (30).

12. Appareil selon l'une des revendications 1 à 11, dans lequel les filaments chauffants forment des sous-ensembles résistifs chauffants (33', 33") connectés électriquement en parallèle.

13. Appareil selon l'une des revendications précédentes, dans lequel :
- la cuve de mesure (3) se présente sous la forme d'un tube ouvert à ses deux extrémités (3a, 3b),
- le moyen de mesure comporte :
∘ un émetteur (34) de rayonnement infrarouge monté au niveau d'une extrémité (3a) de la cuve de mesure (3) de manière à ce que ladite cuve soit traversée par un rayonnement infrarouge,
∘ un détecteur de rayonnement infrarouge (35) monté à l'autre extrémité (3b) de la cuve de mesure (3),
- une cavité étanche au fluide d'haleine est interposée entre l'émetteur (34) de rayonnement infrarouge et l'extrémité correspondante (3a) de la cuve de mesure (3),
- une cavité étanche au fluide d'haleine est interposée entre le détecteur de rayonnement infrarouge (35) et l'extrémité correspondante (3b) de la cuve de mesure (3).

14. Procédé de régulation de la température de la cuve de mesure (3) de l'appareil selon l'une des revendications 1 à 13, consistant à réguler l'énergie électrique injectée dans l'élément résistif chauffant (33) grâce à une boucle de contre-réaction basée sur : la mesure en temps réel de la résistance dudit élément et l'objectif d'atteindre une résistance de consigne correspondant à une température de chauffage cible.

15. Procédé d'utilisation d'un appareil conforme à l'une des revendications 1 à 13, comprenant les étapes consistant à:
- enregistrer et associer, dans une base de données, un moyen d'identification de l'appareil (A) et un moyen d'identification d'un utilisateur,
- préalablement à la mesure, acquérir, depuis un terminal mobile (T) de l'utilisateur, le moyen d'identification de l'appareil (A) et le moyen d'identification de l'utilisateur,
∘ l'acquisition du moyen d'identification de l'utilisateur est basée sur la mise en œuvre d'un algorithme de reconnaissance faciale dudit utilisateur.
∘ l'acquisition du moyen d'identification de l'appareil (A) est basée sur la mise en œuvre d'un algorithme de reconnaissance de la forme dudit appareil ou sur la mise en œuvre d'un algorithme de reconnaissance d'un marquage apposé sur ledit appareil.
- analyser le moyen d'identification de l'appareil (A) acquis et le moyen d'identification de l'utilisateur acquis,
- seulement en cas de correspondance entre le moyen d'identification de l'appareil acquis et le moyen d'identification de l'utilisateur acquis, transmettre à l'unité de commande (9) une instruction pour opérer la mesure, laquelle instruction est générée depuis le terminal mobile (T).

## Patentansprüche

1. Tragbare Einrichtung zur Messung der Konzentration mindestens einer Komponente in einem durch ein Atemfluid ausgeatmeten Gas, umfassend:
- ein Mundstück (1a), durch welches das Atemfluid ausgeatmet wird,
- ein Gehäuse (2), beinhaltend:
∘ einen Messbehälter (3),
∘ ein Messmittel (34, 35), das zur Messung der Konzentration mindestens einer Komponente in einem Gas des in dem Messbehälter (3) zirkulierenden Atemfluids geeignet ist,
∘ eine Öffnung (21A), in der das Mundstück (1a) installiert ist,
- eine erste Kammer (101a, 101b, 101c), die stromaufwärts des Messbehälters (3) angeordnet ist und eine Eintrittsöffnung (100, 100b, 100c) umfassend, durch die das ausgeatmete Atemfluid in die erste Kammer eindringt,
- eine zweite Kammer (110a, 110b, 110c), die stromaufwärts des Messbehälters (3) angeordnet ist, wobei die zweite Kammer aufweist:
∘ eine Eintrittsöffnung (111a, 111b, 111c), die in die erste Kammer (101a, 101b, 101c) mündet,
∘ eine Austrittsöffnung (112a, 111b, 112c) in fluidischer Verbindung mit dem Messbehälter (3) und durch die ein Teil des ausgeatmeten Atemfluids hindurchtritt,
- eine Austrittsöffnung (122a, 122b, 222), durch die ein Teil des ausgeatmeten Atemfluids ausgestoßen wird,
wobei in der Einrichtung:
- die zweite Kammer (110a, 110b, 110c) innerhalb der ersten Kammer (101a, 101b, 101c) oder in einer zu der ersten Kammer benachbarten Position angeordnet ist,
- die Austrittsöffnung (122a, 122b, 222) in die erste Kammer (101a, 101b, 101c) mündet, sodass nur ein Teil des in der ersten Kammer zirkulierenden ausgeatmeten Atemfluids durch die Eintrittsöffnung (111a, 111b, 111c) der zweiten Kammer in die zweite Kammer (110a, 110b, 110c) eindringt, wobei der andere Teil des ausgeatmeten Atemfluids ausgestoßen wird,
die Einrichtung **dadurch gekennzeichnet, dass:**
- die Austrittsöffnung (122a, 122b, 222) eine Austrittsöffnung zur Umgebungsluft ist,
- der Messbehälter (3) aus einem flexiblen, biegsamen Träger (30) ausgebildet ist, der in Form eines Rohres geformt ist,
- der flexible, biegsame Träger (30) eine erste Fläche (30a) und eine zweite Fläche (30b) aufweist, wobei die Flächen einander gegenüberliegen,
- die erste Fläche (30a) mit einem reflektierenden metallischen Material bedeckt ist, das eine optische Reflexionsschicht bildet,
- der flexible, biegsame Träger (30) ein Heizwiderstandselement (33) beinhaltet, das in Form einer flexiblen elektrischen Schaltung ausgestaltet ist, in die ein oder mehrere Heizdrähte in Form von Metallbändern mit einer Dicke von 1 µm bis 50 µm integriert sind, die auf der zweiten Fläche (30b) angeordnet sind.

2. Einrichtung nach Anspruch 1, wobei:
- die erste Kammer (101a) und die zweite Kammer (110a) in dem Mundstück (1a) ausgebildet sind,
oder
- die erste Kammer (101c) und die zweite Kammer (110c) in dem Gehäuse (2) ausgebildet sind,
oder
- der Messbehälter (3), das Messmittel (34, 35), ein Pumpmittel (8), das geeignet ist, das im Messbehälter zirkulierende Atemfluid zu extrahieren, und die Steuereinheit (9) auf einem gemeinsamen Träger (4b) zusammengefasst sind, sodass sie eine greifbare Einheitsanordnung (E) bilden, wobei die Anordnung abnehmbar in einer Aufnahme des Gehäuses (2) installiert ist, wobei die erste Kammer (101b) und die zweite Kammer (110b) in dem gemeinsamen Träger (4b) ausgebildet sind.

3. Einrichtung nach einem der vorstehenden Ansprüche,
umfassend mindestens eines der folgenden Merkmale:
- die zweite Kammer (110a, 110b, 110c) weist reduzierte Abmessungen im Verhältnis zu jenen der ersten Kammer (101a, 101b, 101c) auf,
- die Austrittsöffnung (122a, 122b, 222) zur Umgebungsluft ist so dimensioniert, dass 80 % bis 98 % des in die erste Kammer (101a, 101b, 101c) ausgeatmeten Atemfluids in die Umgebungsluft ausgestoßen wird.

4. Einrichtung nach einem der vorstehenden Ansprüche, wobei:
- der Messbehälter (3), das Messmittel (34, 35), ein Pumpmittel (8) und die Steuereinheit (9) auf einem gemeinsamen Träger (4a, 4b) zusammengefasst sind, sodass sie eine greifbare Einheitsanordnung (E) bilden, wobei die Anordnung abnehmbar in einer Aufnahme des Gehäuses (2) installiert ist,
- das Gehäuse (2) aus mindestens zwei länglichen Rohren (2A, 2B) gebildet ist, die eine gemeinsame Längsachse (X-X) aufweisen, wobei sich diese Rohre entlang der Längsachse ineinanderfügen, um die Aufnahme zu definieren,
- die Einheitsanordnung (E) in einem der Rohre (2A) installiert ist, wobei dieses Rohr (2A) einen Mundstückhalter bildet, in den das Mundstück (1a, 1b) eingesteckt wird,
- das andere Rohr (2B), welches das Gehäuse (2) bildet, geeignet ist, eine elektrische Batterie aufzunehmen, die geeignet ist, die greifbare Einheitsanordnung (E) zu versorgen.

5. Einrichtung nach Anspruch 4,
aufweisend mindestens eines der folgenden Merkmale:
- den gemeinsamen Träger (4a, 4b), der geeignet ist, eine fluidische Verbindung zwischen dem Messbehälter (3) und der Austrittsöffnung (112a, 411b, 112c) der zweiten Kammer (110a, 110b, 110c) zu gewährleisten,
- der gemeinsame Träger (4a, 4b) aufweist:
- eine Aufnahme (43), in der der Messbehälter (3) installiert ist,
- mindestens eine Aufnahme (44, 45), in der das Messmittel (34, 35) installiert ist,
- eine Aufnahme (48), in der das Pumpmittel (8) installiert ist,
- eine oder mehrere Baugruppen (900), die geeignet sind, die Steuereinheit (9) aufzunehmen.

6. Einrichtung nach einem der vorstehenden Ansprüche, wobei:
- der Messbehälter (3), das Messmittel (34, 35), ein Pumpmittel (8) und die Steuereinheit (9) auf einem gemeinsamen Träger (4a, 4b) zusammengefasst sind, um eine greifbare Einheitsanordnung (E) zu bilden, wobei die Anordnung abnehmbar in der Aufnahme des Gehäuses (2) installiert ist,
- der gemeinsame Träger (4a, 4b) aufweist:
∘ eine erste Bohrung (410), die in eine Aufnahme (43) mündet, in der der Messbehälter (3) installiert ist, sodass die Bohrung in fluidischer Verbindung mit dem Behälter steht,
∘ eine zweite Bohrung (420), die in eine in dem Träger angeordnete Kammer (42) mündet, in der ein Drucksensor installiert ist,
- die Austrittsöffnung (112a, 411b, 112c) der zweiten Kammer (110a, 110b, 110c) in fluidischer Verbindung mit der ersten Bohrung (410) und mit der zweiten Bohrung (420) steht,
- die erste Bohrung (410) konisch ist, wobei die erste Bohrung eine erste Öffnung (411) und eine zweite Öffnung (412) aufweist, die in das Gehäuse (43) mündet, in dem der Messbehälter (3) installiert ist, wobei der Durchmesser der ersten Öffnung (411) kleiner ist als der Durchmesser der zweiten Öffnung (412).

7. Einrichtung nach einem der vorstehenden Ansprüche, wobei:
- die Eintrittsöffnung (100, 100b, 100c) der ersten Kammer (101a, 101b, 101c), die Eintrittsöffnung (111a, 111b, 111c) der zweiten Kammer (110a, 110b, 110c) und die Austrittsöffnung (112a, 411b, 112c) der zweiten Kammer in derselben Ausrichtung (Y-Y) angeordnet sind,
- die Austrittsöffnung (122a, 112b, 222) zur Umgebungsluft in einer Richtung (Z-Z) ausgerichtet ist, die senkrecht zu dieser Ausrichtung (Y-Y) steht.

8. Einrichtung nach einem der vorstehenden Ansprüche
in Kombination mit Anspruch 5,
wobei die Aufnahme (48), in der das Pumpmittel (8) installiert ist, eine Bohrung (480) aufweist, die in die Aufnahme (43) mündet, in der der Messbehälter (3) installiert ist, sodass die Aufnahmen (43, 48) in fluidischer Verbindung stehen.

9. Einrichtung nach einem der vorstehenden Ansprüche, wobei:
- der Messbehälter (3) und das Gehäuse (2) jeweils eine Längsachse (X-X; X'-X') aufweisen, wobei die Achsen parallel sind,
- ein Pumpmittel (8) konfiguriert ist, um das in dem Messbehälter (3) zirkulierende Atemfluid in einer Richtung parallel zu den Längsachsen (X-X; X'-X') auszustoßen.

10. Einrichtung nach einem der vorstehenden Ansprüche, wobei:
- der flexible, biegsame Träger (30) zwei gegenüberliegende Längskanten (32a, 32b) aufweist, die durch Kleben miteinander verbunden sind, um die Form des Trägers in Rohrform aufrechtzuerhalten,
∘ eine der Kanten (32a) einen Streifen (320) ohne Heizwiderstandselement (33) aufweist.

11. Einrichtung nach einem der vorstehenden Ansprüche, wobei:
- der oder die Heizfäden den flexiblen Träger (30) gleichmäßig bedecken, sodass die vom Heizwiderstandselement (33) entwickelte elektrische Leistungsdichte auf der gesamten zweiten Seite (30b) des Trägers (30) identisch ist,
oder
- der oder die Heizfäden den flexiblen Träger (30) ungleichmäßig bedecken, sodass die vom Heizwiderstandselement (33) entwickelte elektrische Leistungsdichte entlang einer Längsachse (X"-X") und/oder einer Querachse (Y"-Y") des flexiblen Trägers (30) variiert.

12. Einrichtung nach einem der Ansprüche 1 bis 11, wobei die Heizfäden elektrisch parallel geschaltete Heizwiderstandsunteranordnungen (33', 33") bilden.

13. Einrichtung nach einem der vorstehenden Ansprüche, wobei:
- der Messbehälter (3) die Form eines an seinen beiden Enden (3a, 3b) offenen Rohres aufweist,
- das Messmittel aufweist:
∘ einen Infrarotstrahlungsemitter (34), der an einem Ende (3a) des Messbehälters (3) angebracht ist, sodass der Messbehälter von einer Infrarotstrahlung durchdrungen wird,
∘ einen Infrarotstrahlungsdetektor (35), der am anderen Ende (3b) des Messbehälters (3) angebracht ist,
- zwischen dem Infrarotstrahlungsemitter (34) und dem entsprechenden Ende (3a) des Messbehälters (3) ein gegen Atemluft abgedichteter Hohlraum angeordnet ist,
- zwischen dem Infrarotstrahlungsdetektor (35) und dem entsprechenden Ende (3b) des Messbehälters (3) ein gegen Atemluft abgedichteter Hohlraum angeordnet ist.

14. Verfahren zur Regelung der Temperatur des Messbehälters (3) der Einrichtung nach einem der Ansprüche 1 bis 13, bestehend aus der Regelung der in das Heizwiderstandselement (33) eingespeisten elektrischen Energie mittels einer Rückkopplungsschleife, basierend auf: der Echtzeitmessung des Widerstands des Elements und dem Ziel, einen Sollwiderstand zu erreichen, der einer Sollheiztemperatur entspricht.

15. Verfahren zur Verwendung einer Einrichtung
nach einem der Ansprüche 1 bis 13, umfassend die Schritte bestehend aus:
- Speichern und Verknüpfen eines Identifikationsmittels für die Einrichtung (A) und eines Identifikationsmittels für einen Benutzer in einer Datenbank,
- vor der Messung über ein mobiles Endgerät (T) des Benutzers, Erfassen der Identifikationsdaten der Einrichtung (A) und der Identifikationsdaten des Benutzers,
∘ Erfassen des Identifikationsmittels für den Benutzer basierend auf der Anwendung eines Algorithmus zur Gesichtserkennung des Benutzers.
∘ Erfassen des Identifikationsmittels für die Einrichtung (A) basierend auf der Anwendung eines Algorithmus zur Erkennung der Form der Einrichtung oder auf der Anwendung eines Algorithmus zur Erkennung einer auf der Einrichtung angebrachten Kennzeichnung.
- Analysieren des erfassten Identifikationsmittels für die Einrichtung (A) und des erfassten Identifikationsmittels für den Benutzer,
- nur wenn das erfasste Identifikationsmittel für die Einrichtung mit dem erfassten Identifikationsmittel für den Benutzer übereinstimmen, Übermitteln einer Anweisung zum Durchführen der Messung an die Steuereinheit (9), wobei diese Anweisung vom mobilen Endgerät (T) generiert wird.

## Claims

1. Portable apparatus for measuring the concentration of at least one component in a gas exhaled by a respiratory fluid, comprising:
- a mouthpiece (1a) through which the respiratory fluid is exhaled,
- a housing (2) integrating:
∘ a measuring vessel (3),
∘ a measuring means (34, 35) for measuring the concentration of at least one component in a gas of the respiratory fluid circulating in the measuring vessel (3),
∘ an opening (21A) in which the mouthpiece (1a) is installed,
- a first chamber (101a, 101b, 101c) located upstream of the measuring vessel (3) and comprising an inlet orifice (100, 100b, 100c) through which the exhaled respiratory fluid enters said first chamber,
- a second chamber (110a, 110b, 110c) located upstream of the measuring vessel (3), which second chamber comprises:
∘ an inlet orifice (111a, 111b, 111c) opening into the first chamber (101a, 101b, 101c),
∘ an outlet orifice (112a, 111b, 112c) which is in fluid communication with the measuring vessel (3) and through which one part of the exhaled respiratory fluid passes,
- an outlet orifice (122a, 122b, 222) through which one part of the exhaled respiratory fluid is expelled,
wherein:
- the second chamber (110a, 110b, 110c) is arranged inside the first chamber (101a, 101b, 101c) or in a position adjacent to said first chamber,
- the outlet orifice (122a, 122b, 222) opens into the first chamber (101a, 101b, 101c) so that only one part of the exhaled respiratory fluid circulating in said first chamber enters the second chamber (110a, 110b, 110c) via the inlet orifice (111a, 111b, 111c) of said second chamber, the other part of the exhaled respiratory fluid being expelled,
**characterized in that:**
- the outlet orifice (122a, 122b, 222) is an outlet orifice to the ambient air,
- the measuring vessel (3) is made from a flexible non-rigid support (30) in the form of a tube,
- the flexible non-rigid support (30) comprises a first face (30a) and a second face (30b), which faces are opposite one another,
- the first face (30a) is coated with a reflective metal material forming an optical reflection layer,
- the flexible non-rigid support (30) incorporates a resistive heating element (33), which heating element is in the form of a flexible electrical circuit into which one or more heating filaments in the form of metal strips are integrated, which strips have a thickness of 1 µm to 50 µm and are arranged on the second face (30b).

2. Apparatus according to claim 1, wherein:
- the first chamber (101a) and the second chamber (110a) are formed in the mouthpiece (1a),
or
- the first chamber (101c) and the second chamber (110c) are formed in the housing (2),
or
- the measuring vessel (3), the measuring means (34, 35), a pumping means (8) for extracting the respiratory fluid circulating in the measuring vessel, and the control unit (9) are combined on a common support (4b) so as to form a graspable single assembly (E), which assembly is removably installed in a recess of the housing (2), the first chamber (101b) and the second chamber (110b) being formed in said common support (4b).

3. Apparatus according to either of the preceding claims, comprising at least one of the following features:
- the second chamber (110a, 110b, 110c) is smaller than the first chamber (101a, 101b, 101c).
- the outlet orifice (122a, 122b, 222) to the ambient air is sized so that 80% to 98% of the respiratory fluid exhaled in the first chamber (101a, 101b, 101c) is expelled to the ambient air.

4. Apparatus according to any of the preceding claims, wherein:
- the measuring vessel (3), the measuring means (34, 35), a pumping means (8) and the control unit (9) are combined on a common support (4a, 4b) so as to form a graspable single assembly (E), which assembly is removably installed in a recess of the housing (2),
- the housing (2) is made of at least two elongate tubes (2A, 2B) having a common longitudinal axis (X-X), which tubes fit together along said longitudinal axis to define the recess,
- the single assembly (E) is installed in one of the tubes (2A), this tube (2A) forming a mouthpiece-holder into which the mouthpiece (1a, 1b) fits,
- the other tube (2B) forming the housing (2) is suitable for receiving an electric battery for powering the graspable single assembly (E).

5. Apparatus according to claim 4, comprising at least one of the following features:
- the common support (4a, 4b) is suitable for providing fluid communication between the measuring vessel (3) and the outlet orifice (112a, 411 b, 112c) of the second chamber (110a, 110b, 110c),
- the common support (4a, 4b) comprises:
- a recess (43) in which the measuring vessel (3) is installed,
- at least one recess (44, 45) in which the measuring means (34, 35) is installed,
- a recess (48) in which the pumping means (8) is installed,
- one or more fittings (900) for receiving the control unit (9).

6. Apparatus according to any of the preceding claims, wherein:
- the measuring vessel (3), the measuring means (34, 35), the pumping means (8) and the control unit (9) are combined on a common support (4a, 4b) so as to form a graspable single assembly (E), which assembly is removably installed in the recess of the housing (2),
- the common support (4a, 4b) comprises:
∘ a first bore (410) which opens into a recess (43) in which the measuring vessel (3) is installed so that said bore is in fluid communication with said vessel,
∘ a second bore (420) which opens into a chamber (42) which is arranged in said support and in which a pressure sensor is installed,
- the outlet orifice (112a, 411 b, 112c) of the second chamber (110a, 110b, 110c) is in fluid communication with the first bore (410) and with the second bore (420),
- the first bore (410) is conical, which first bore comprises a first orifice (411) and a second orifice (412) which opens into the recess (43) in which the measuring vessel (3) is installed, the diameter of said first orifice (411) being smaller than the diameter of said second orifice (412).

7. Apparatus according to any of the preceding claims, wherein:
- the inlet orifice (100, 100b, 100c) of the first chamber (101 a, 101 b, 101c), the inlet orifice (111 a, 111 b, 111c) of the second chamber (110a, 110b, 110c) and the outlet orifice (112a, 411b, 112c) of said second chamber are arranged in the same alignment (Y-Y),
- the outlet orifice (122a, 112b, 222) to the ambient air is oriented in a direction (Z-Z) which is perpendicular to this alignment (Y-Y).

8. Apparatus according to any of the preceding claims in combination with claim 5, wherein the recess (48) in which the pumping means (8) is installed comprises a bore (480) opening into the recess (43) in which the measuring vessel (3) is installed, such that said recesses (43, 48) are in fluid communication.

9. Apparatus according to any of the preceding claims, wherein:
- the measuring vessel (3) and the housing (2) each have a longitudinal axis (X-X; X'-X'), which axes are parallel,
- a pumping means (8) is designed to expel the respiratory fluid circulating in the measuring vessel (3) in a direction which is parallel to said longitudinal axes (X-X; X'-X').

10. Apparatus according to any of the preceding claims, wherein:
- the flexible non-rigid support (30) comprises two opposed longitudinal edges (32a, 32b) which are rigidly connected to one another by gluing in order to maintain the shape of said support in the form of a tube,
∘ one of said edges (32a) has a strip (320) without a resistive heating element (33).

11. Apparatus according to any of the preceding claims, wherein:
- the heating filament(s) cover(s) the flexible non-rigid support (30) homogeneously so that the electrical power density developed by the resistive heating element (33) is identical across the entire second face (30b) of said support (30), or
- the heating filament(s) cover(s) the flexible non-rigid support (30) inhomogeneously so that the electrical power density developed by the resistive heating element (33) varies along a longitudinal axis (X"-X") and/or along a transverse axis (Y"-Y") of the flexible non-rigid support (30).

12. Apparatus according to any of claims 1 to 11, wherein the heating filaments form resistive heating sub-assemblies (33', 33") electrically connected in parallel.

13. Apparatus according to any of the preceding claims, wherein:
- the measuring vessel (3) is in the form of a tube open at both ends (3a, 3b),
- the measuring means comprises:
∘ an infrared radiation transmitter (34) mounted at one end (3a) of the measuring vessel (3) so that infrared radiation passes through said vessel,
∘ an infrared radiation detector (35) mounted at the other end (3b) of the measuring vessel (3),
- a respiratory-fluid-tight cavity is positioned between the infrared radiation transmitter (34) and the corresponding end (3a) of the measuring vessel (3),
- a respiratory-fluid-tight cavity is positioned between the infrared radiation detector (35) and the corresponding end (3b) of the measuring vessel (3).

14. Method for regulating the temperature of the measuring vessel (3) of the apparatus according to any of claims 1 to 13, consisting in regulating the electrical energy injected into the resistive heating element (33), by means of a negative feedback loop based on: real-time measurement of the resistance of said element and the objective of reaching a set resistance corresponding to a target heating temperature.

15. Method for using an apparatus according to any of claims 1 to 13, comprising the steps of:
- storing and associating, in a database, a means for identifying the apparatus (A) and a means for identifying a user,
- prior to the measurement, acquiring, from a mobile terminal (T) of the user, the means for identifying the apparatus (A) and the means for identifying the user,
∘ the acquisition of the means for identifying the user is based on using an algorithm for facial recognition of said user,
∘ the acquisition of the means for identifying the apparatus (A) is based on using an algorithm for recognizing the shape of said apparatus or based on using an algorithm for recognizing a marking affixed to said apparatus,
- analyzing the acquired means for identifying the apparatus (A) and the acquired means for identifying the user,
- conveying to the control unit (9) an instruction to perform the measurement, which instruction is generated from the mobile terminal (T), only if there is a correspondence between the acquired means for identifying the apparatus and the acquired means for identifying the user.
